# EUROPÄISCHE PATENTSCHRIFT

(11) **EP 3 757 094 B1**
(45) Veröffentlichungstag und Bekanntmachung des Hinweises auf die Patenterteilung: **11.10.2023**
(21) Anmeldenummer: 20183460.3
(22) Anmeldetag: 21.06.2016
(51) Int. Cl.: C07D 239/06, C08F 2/50

(54) **AMIDIN-KATALYSATOR FÜR HÄRTBARE ZUSAMMENSETZUNGEN**
AMIDINE CATALYST FOR CURABLE COMPOSITIONS
CATALYSEUR DE TYPE AMIDINE POUR COMPOSITIONS DURCISSABLES

(30) Priorität: 22.06.2015 EP 15173193
(43) Veröffentlichungstag der Anmeldung: 30.12.2020
(62) Teilanmeldung aus: 16733369.9
(73) Patentinhaber: SIKA TECHNOLOGY AG, 6340 Baar (CH)
(72) Erfinder: Cannas, Rita, 8600 Dübendorf (CH); Burckhardt, Urs, 8049 Zürich (CH)
(74) Vertreter: Sika Patent Attorneys

(56) Entgegenhaltungen:
- WO-A1-97/31033
- CN-A- 101 747 826
- FR-A1- 2 144 724
- US-A- 2 994 685
- US-A1- 2009 029 888
- BI-ZHOU LIN ET AL: "Hydrothermal Synthesis and Characterization of Two New Phosphatomolybdates(V) Containing Sandwich-Shaped [M(Mo6P4)2] Clusters (M = Co, Ni)", JOURNAL OF CLUSTER SCIENCE, KLUWER ACADEMIC PUBLISHERS-PLENUM PUBLISHERS, NE, Bd. 19, Nr. 2, 3. Januar 2008 (2008-01-03) , Seiten 379-390, XP019608273, ISSN: 1572-8862
- ISAGULYANTS, V.I., ET AL: "structure and tautomerism of substituted imidazolines", ZHURNAL PRIKLKADNOI KHIMII, Bd. 41, Nr. 7, 1968, Seiten 1585-1590, XP009186249,

## Beschreibung

### Technisches Gebiet

Die Erfindung betrifft Amidine und ihre Verwendung als Katalysatoren für härtbare Zusammensetzungen.

### Stand der Technik

Härtbare Zusammensetzungen spielen eine bedeutende Rolle in vielen technischen Anwendungen, beispielsweise als Klebstoffe, Dichtstoffe oder Beschichtungen. Ihre Aushärtung wird durch Vernetzungsreaktionen bewirkt, welche über freie oder latente Reaktivgruppen wie beispielsweise Isocyanatgruppen, Epoxidgruppen, Hydroxylgruppen, Aminogruppen oder Silangruppen ablaufen, wobei diese nach einem Mischvorgang, durch Erwärmen oder durch Kontakt mit Feuchtigkeit mit sich selbst oder untereinander reagieren und so die in der Zusammensetzung enthaltenen Aufbaukomponenten kovalent zu einem polymeren Netzwerk verbinden. Zur Beschleunigung solcher Vernetzungsreaktionen werden häufig Katalysatoren eingesetzt. Sehr oft handelt es sich dabei um toxikologisch bedenkliche Stoffe, welche eine potentielle Gefährdung von Verarbeiter und Umwelt darstellen, insbesondere nach der Aushärtung der Zusammensetzung, wenn der Katalysator oder dessen Abbauprodukte durch Ausgasen, Migration oder Auswaschen freigesetzt werden.

Bei Raumtemperatur härtbare Zusammensetzungen auf Basis von Silangruppen-haltigen Polymeren sind ausgeprägt mit dieser Problemstellung konfrontiert. Silangruppen-haltige Polymere sind dabei insbesondere Polyorganosiloxane, welche gemeinhin als "Silicone" bzw. "Siliconkautschuke" bezeichnet werden, und Silangruppen-haltige organische Polymere, welche auch als "silanfunktionelle Polymere", "silanmodifizierte Polymere" (SMP) oder "silanterminierte Polymere" (STP) bezeichnet werden. Ihre Vernetzung verläuft über die Kondensation von Silanolgruppen unter Ausbildung von Siloxanbindungen und wird klassischerweise mittels Organozinn-Verbindungen, wie insbesondere Di-alkylzinn(IV)-carboxylaten, katalysiert. Diese zeichnen sich durch eine sehr hohe Aktivität in Bezug auf die Silanol-Kondensation aus und sind sehr hydrolysebeständig; allerdings sind sie gesundheitsschädlich und stark wassergefährdend. Oft werden sie mit weiteren Katalysatoren kombiniert, hauptsächlich mit basischen Verbindungen wie insbesondere Aminen, welche vor allem die vorgeschaltete Hydrolyse der Silangruppen beschleunigen.

Aufgrund einer stärkeren Gewichtung von EHS-Aspekten durch Berufsverbände und Verbraucher sowie schärferer staatlicher Regulierung werden seit einiger Zeit vermehrt Anstrengungen unternommen, die Organozinn-Verbindungen durch andere, weniger toxische Katalysatoren zu ersetzen. So wurden etwa Organotitanate, -zirkonate und -aluminate als alternative Metall-Katalysatoren beschrieben. Diese haben aber zumeist eine geringere katalytische Aktivität in Bezug auf die Silanol-Kondensation und bewirken eine deutlich langsamere Vernetzung. Wegen ihrer mangelnden Hydrolysestabilität können sie beim Lagern der Zusammensetzung durch Restfeuchte der Inhaltsstoffe einen Grossteil ihrer Aktivität verlieren, wodurch sich die Aushärtung stark verlangsamt oder ganz zum Erliegen kommt.

Eine weitere bekannte Alternative zu Organozinn-Verbindungen stellen stark basische Stickstoff-Verbindungen aus der Klasse der Amidine und Guanidine dar, welche in Kombination mit den erwähnten Metall-Katalysatoren oder auch allein eingesetzt werden können. Viele der gebräuchlichen Amidin- und Guanidin-Katalysatoren, wie insbesondere 1,8-Diazabicyclo[5.4.0]undec-7-en (DBU) und 1,1,3,3-Tetramethylguanidin (TMG), sind allerdings leichtflüchtige und geruchsintensive sowie ebenfalls gesundheitsschädliche und umweltgefährdende Stoffe. Ausserdem neigen sie dazu, aufgrund geringer Verträglichkeit in der Zusammensetzung zu migrieren und dadurch Separation, Ausschwitzen oder Substratverschmutzung zu verursachen. Die beschriebene Verwendung von aromatischen, bei Raumtemperatur festen Amidinen und Guanidinen schafft hier Abhilfe, erfordert aber den Einsatz von geeigneten Lösemitteln und bringt Einbussen bei der katalytischen Aktivität und damit der Vernetzungsgeschwindigkeit.

In den Publikationen US 2009/029888 A1, CN 101747826 A, FR 2144724 A1, WO 97/31033 A1 sowie US 2994685 A werden verschiedene Aminderivate wie beispielsweise Derivate von Imidazolen, Amidinen oder Guanidinen offenbart, welche teilweise als Epoxyhärter eingesetzt werden. Deren Einsatz als Katalysatoren für die Vernetzung von insbesondere Silangruppen-haltigen Zusammensetzungen wird jedoch nicht gelehrt.

### Darstellung der Erfindung

Aufgabe der vorliegenden Erfindung ist es daher, einen Katalysator für die Vernetzung von härtbaren Zusammensetzungen, insbesondere Silangruppen-haltigen Zusammensetzungen, bereitzustellen, welcher eine hohe katalytische Aktivität für die Vernetzungsreaktion besitzt und damit eine rasche Aushärtung der applizierten Zusammensetzung ermöglicht, sowie eine hohe Selektivität für diese Vernetzungsreaktion aufweist und somit die Lagerstabilität der Zusammensetzung nicht über Gebühr beeinträchtigt. Weiterhin soll der Katalysator einen geringen Dampfdruck und eine hohe Verträglichkeit mit der Zusammensetzung aufweisen, so dass er weder zu Separierung oder Migration noch zum Verdampfen neigt, und soll möglichst geruchlos und wenig toxisch sein.

Diese Aufgabe wird durch ein Amidin der Formel (I) wie in Anspruch 1 beschrieben gelöst. Das Amidin der Formel (I) enthält mindestens eine aliphatische Amidin-Gruppe. Es ist nur wenig oder gar nicht flüchtig und zeigt eine hohe Aktivität bei der Verwendung als Katalysator für härtbare Zusammensetzungen, während aromatische Amidine kaum oder gar nicht katalytisch aktiv sind. Im Gegensatz zu vielen aus dem Stand der Technik bekannten aliphatische Amidin- oder Guanidin-Gruppen aufweisenden Katalysatoren ist das Amidin der Formel (I) bei Raumtemperatur weitgehend geruchlos und nicht flüchtig. Er zeigt eine hohe kalatytische Aktivität bei guter Selektivität, insbesondere in Zusammensetzungen auf Basis von Silangruppen-haltigen Polymeren. Dies ist besonders überraschend, würde man doch aufgrund seines relativ hohen Molekulargewichtes und der starken intermolekularen Wechselwirkungen über Wasserstoffbrücken eine verminderte Aktivität im Vergleich mit kleineren, unpolareren und somit mobileren Amidinen erwarten.

Mit diesen Eigenschaften ist das Amidin der Formel (I) besonders geeignet für die Verwendung in Zusammensetzungen auf Basis von Silangruppen-haltigen Polymeren, wo es als alleiniger Katalysator oder in Kombination mit weiteren Katalysatoren eine schnelle Aushärtung zu einem mechanisch hochwertigen und beständigen Material ermöglicht, ohne die Lagerfähigkeit der unausgehärteten Zusammensetzung zu beeinträchtigen. Es ist sowohl vor als auch nach der Aushärtung ausgezeichnet verträglich mit der Zusammensetzung und neigt weder zu Separierung noch zu Migration, im Gegensatz zu vielen ähnlichen Zusammensetzungen mit Amidin- oder Guanidin-Katalysatoren nach dem Stand der Technik, wo Katalysator-bedingte Migrationseffekte eine starke Rolle spielen. Es ermöglicht emissions- und geruchsarme Produkte, welche weder schmierige oder klebrige Oberflächen aufweisen noch Substratverschmutzung verursachen. Schliesslich ist das Amidin der Formel (I) in einem überraschend einfachen Verfahren ohne Hilfsstoffe aus handelsüblichen, preisgünstigen Ausgangsmaterialien herstellbar.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist ein Amidin der Formel (I), 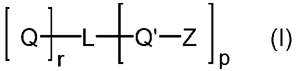 wobei
p für 1 und r für 0 steht,
L für einen mit einer Alkoxysilangruppe substituierten Alkylrest mit 1 bis 6 C-Atomen steht,
Q für Glycidoxy steht,
Q' für eine zweiwertige Verbindungseinheit entstanden aus der Reaktion einer Reaktivgruppe Q mit HZ steht, wobei
Q'für 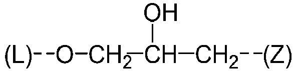 steht, und
Z für eine über ein Stickstoffatom gebundene aliphatische Amidingruppe steht.

Im vorliegenden Dokument bezeichnet der Begriff "aliphatische Amidingruppe" eine Amidingruppe, welche kein Stickstoffatom enthält, welches direkt an einen aromatischen Ring gebunden oder Teil eines heteroaromatischen Ringsystems, wie zum Beispiel Imidazol oder Pyrimidin, ist.

Als "primäre Aminogruppe" bzw. "primärer Amin-Stickstoff" wird eine NH₂-Gruppe bzw. deren Stickstoffatom bezeichnet, die bzw. das an einen organischen Rest gebunden ist, und als "sekundäre Aminogruppe" bzw. "sekundärer Amin-Stickstoff" wird eine NH-Gruppe bzw. deren Stickstoffatom bezeichnet, die bzw. das an zwei organische Reste, welche auch gemeinsam Teil eines Rings sein können, gebunden ist, und als "tertiäre Aminogruppe" bzw. "tertiärer Amin-Stickstoff" wird eine N-Gruppe bzw. deren Stickstoffatom bezeichnet, die bzw. das an drei organische Reste, welche auch zu zweit oder zu dritt Teil eines oder mehrerer Ringe sein können, gebunden ist.

Der Begriff "Silangruppe" bezeichnet eine an einen organischen Rest oder an einen Polyorganosiloxan-Rest gebundene Silylgruppe mit einer bis drei, insbesondere zwei oder drei, hydrolysierbaren Substituenten am Silicium-Atom. Besonders gebräuchliche hydrolysierbare Substituenten sind Alkoxy-Reste. Diese Silangruppen werden auch als "Alkoxysilangruppen" bezeichnet. Silangruppen können auch in partiell oder vollständig hydrolysierter Form vorhanden sein.

Als "Hydroxysilan", "Isocyanatosilan", "Aminosilan" bzw. "Mercaptosilan" werden Organoalkoxysilane bezeichnet, die am organischen Rest zusätzlich zur Silangruppe eine oder mehrere Hydroxyl-, Isocyanato-, Amino- bzw. Mercaptogruppen aufweisen.

Mit "Poly" beginnende Substanznamen wie Polyol oder Polyisocyanat bezeichnen Substanzen, die formal zwei oder mehr der in ihrem Namen vorkommenden funktionellen Gruppen pro Molekül enthalten.

Der Begriff "organisches Polymer" umfasst ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde und im PolymerRückgrat mehrheitlich Kohlenstoffatome aufweist, sowie Umsetzungsprodukte eines solchen Kollektivs von Makromolekülen. Polymere mit einem Polyorganosiloxan-Rückgrat (gemeinhin als "Silicone" bezeichnet) stellen keine organischen Polymere im Sinne des vorliegenden Dokuments dar.

Der Begriff "Silangruppen-haltiger Polyether" umfasst auch Silangruppen-haltige organische Polymere, welche zusätzlich zu Polyether-Einheiten auch Urethangruppen, Harnstoffgruppen oder Thiourethangruppen enthalten können. Solche Silangruppen-haltige Polyether können auch als "Silangruppen-haltige Polyurethane" bezeichnet werden.

Unter "Molekulargewicht" versteht man im vorliegenden Dokument die molare Masse (in Gramm pro Mol) eines Moleküls oder eines Teils eines Moleküls, auch als "Rest" bezeichnet. Als "mittleres Molekulargewicht" wird das Zahlenmittel Mₙ einer oligomeren oder polymeren Mischung von Molekülen oder Resten bezeichnet, welches üblicherweise mittels Gelpermeationschromatographie (GPC) gegen Polystyrol als Standard bestimmt wird.

Als "lagerstabil" oder "lagerfähig" wird eine Substanz oder eine Zusammensetzung bezeichnet, wenn sie bei Raumtemperatur in einem geeigneten Gebinde während längerer Zeit, typischerweise mindestens 3 Monaten bis zu 6 Monaten und mehr, aufbewahrt werden kann, ohne dass sie sich in ihren Anwendungs- oder Gebrauchseigenschaften, insbesondere der Viskosität und der Vernetzungsgeschwindigkeit, durch die Lagerung in einem für ihren Gebrauch relevanten Ausmass verändert.

Eine gestrichelte Linie in den Formeln in diesem Dokument stellt jeweils die Bindung zwischen einem Substituenten und dem zugehörigen Molekülrest dar. Als "Raumtemperatur" wird eine Temperatur von ca. 23 °C bezeichnet.

Bevorzugt steht Z für 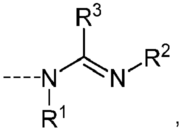 wobei
R¹ für einen Wasserstoff-Rest oder für einen Alkyl- oder Cycloalkyl- oder Aralkyl-Rest mit 1 bis 8 C-Atomen oder zusammen mit R² für R⁴ steht,
R² für einen Wasserstoff-Rest oder für einen Alkyl-, Cycloalkyl- oder Aralkyl-Rest mit 1 bis 18 C-Atomen, welcher gegebenenfalls Ether-Sauerstoff oder tertiären Amin-Stickstoff enthält, oder zusammen mit R¹ für R⁴ steht,
R³ für einen Wasserstoff-Rest oder für einen Alkyl- oder Cycloalkyl- oder Aralkyl-Rest mit 1 bis 12 C-Atomen steht,
wobei R⁴ für einen gegebenenfalls substituierten 1,2-Ethylen-, 1,3-Propylen-oder 1,4-Butylen-Rest mit 2 bis 12 C-Atomen steht,
und wobei
R² und R³ auch zusammen für einen Alkylen-Rest mit 3 bis 6 C-Atomen stehen können.
R¹ steht bevorzugt für einen Alkyl- oder Cycloalkyl- oder Aralkyl-Rest mit 1 bis 4 C-Atomen oder zusammen mit R² für R⁴.
R² steht bevorzugt für einen Alkyl-, Cycloalkyl- oder Aralkyl-Rest mit 1 bis 12, insbesondere 1 bis 8, C-Atomen, welcher gegebenenfalls Ether-Sauerstoff oder tertiären Amin-Stickstoff enthält, oder zusammen mit R¹ für R⁴.
R³ steht bevorzugt für einen Wasserstoff-Rest oder für einen Alkyl-, Cycloalkyl-oder Aralkyl-Rest mit 1 bis 8, insbesondere 1 bis 4, C-Atomen.
R³ steht besonders bevorzugt für einen Wasserstoff-Rest oder für einen Methyl-Rest, am meisten bevorzugt für einen Methyl-Rest.

Besonders bevorzugt stehen R¹ und R² zusammen für R⁴.

Besonders bevorzugt weist Z somit die Formel 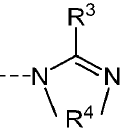 auf.

Ein solches Amidin der Formel (I) ist besonders einfach und in hoher Reinheit herstellbar.

R⁴ weist bevorzugt 2 bis 6 C-Atome auf.

R⁴ steht bevorzugt für 1,2-Ethylen, 1,2-Propylen, 1 ,3-Propylen, 2-Methyl-1 ,2-propylen, 2,2-Dimethyl-1,3-propylen, 1,3-Butylen, 1,4-Butylen, 1,3-Pentylen, 1,2-Cyclohexylen, 1,3-Cyclohexylen oder 2(4)-Methyl-1,3-cyclohexylen.

R⁴ steht besonders bevorzugt für 1,2-Ethylen, 1,2-Propylen, 1,3-Propylen, 2-Methyl-1,2-propylen, 2,2-Dimethyl-1,3-propylen, 1,3-Butylen oder 1,3-Pentylen, insbesondere für 1,2-Ethylen oder 1,3-Propylen.

Am meisten bevorzugt steht R⁴ für 1,3-Propylen.

Besonders bevorzugt stehen R³ für Methyl und R⁴ für 1,3-Propylen. Ein solches Amidin weist eine besonders hohe katalytische Aktivität auf und ist besonders einfach herstellbar.

p steht für 1.

r steht für 0.

Q steht für Glycidoxy.

Dabei steht Glycidoxy für eine Reaktivgruppe der Formel

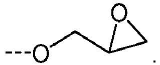

Q' ist

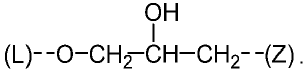

Die in Klammern stehenden Buchstaben (L) und (Z) stellen dabei jeweils die Anbindung von Q' an L und Z dar.

Da Q' für 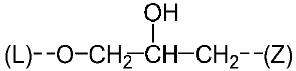 steht, ist Q' entstanden aus der Reaktion von HZ mit einer Glycidoxy-Gruppe.

L steht in diesem Fall bevorzugt für 3-Glycidoxypropyltrimethoxysilan und 3-Glycidoxypropyltriethoxysilan jeweils nach Entfernung der Glycidoxygruppen. Besonders bevorzugt stehen dabei p für 1 und r für 0 und L steht für Triethoxysilylpropyl oder Trimethoxysilylpropyl. Ein solches Amidin der Formel (I) ist besonders geeignet als Katalysator für Silangruppen-haltige Zusammensetzungen, wo es bei der Aushärtung über die Silangruppen kovalent gebunden werden kann.

Die Amidine der Formel (I) sind aus gut zugänglichen Ausgangsstoffen in einem einfachen Verfahren herstellbar und/oder weisen eine besonders hohe katalytische Aktivität auf.

Das Amidin der Formel (I) kann auch in tautomerer Form vorliegen. Alle möglichen Tautomerformen der Amidine werden im Rahmen der vorliegenden Erfindung als gleichwertig angesehen.

Weiterhin kann das Amidin der Formel (I) in protonierter Form vorliegen. Ebenfalls kann das Amidin der Formel (I) in komplexierter Form vorliegen, insbesondere mit Kationen von Zink, Eisen oder Molybdän.

Das Amidin der Formel (I) enthält eine Silangruppe. Dabei stehen p für 1, r für 0, L für einen mit einer Alkoxysilangruppe substituierten Alkylenrest mit 1 bis 6 C-Atomen, und Q' steht für 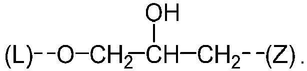 Ein solches Amidin der Formel (I) ist besonders geeignet als Katalysator für Silangruppen-haltige Zusammensetzungen, wo es bei der Aushärtung über die Silangruppen kovalent gebunden werden kann. Es ist insbesondere abgeleitet von einem Glycidoxy- funktionellen Organosilan.

Das Amidin weist dabei insbesondere die Formel (I'), 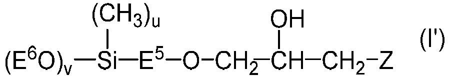 wobei
E⁵ für einen Alkylenrest mit 1 bis 6 C-Atomen, insbesondere für 1,3-Propylen, steht,
E⁶ für einen Alkylrest mit 1 bis 4 C-Atomen, insbesondere für Methyl oder für Ethyl, steht, und
u für 0 oder 1 und v für 2 oder 3 stehen, wobei (u+v) für 3 steht.

Das Amidin der Formel (I) wird bevorzugt erhalten durch die Umsetzung von
- mindestens einem Amidin der Formel HZ, mit
- mindestens einer funktionellen Verbindung der Formel 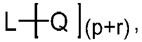
wobei
L, Z, Q, p und r die bereits genannten Bedeutungen aufweisen.

Besonders bevorzugt stehen dabei p für 1 und r für 0 und L steht für Triethoxysilylpropyl oder Trimethoxysilylpropyl. Ein solches Amidin der Formel (I) ist besonders geeignet als Katalysator für Silangruppen-haltige Zusammensetzungen, wo es bei der Aushärtung über die Silangruppen kovalent gebunden werden kann.

Die Umsetzung des Amidins der Formel HZ mit der funktionellen Verbindung der Formel 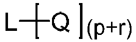 erfolgt insbesondere unter Bedingungen, wie sie für Reaktionen zwischen den an der jeweiligen Umsetzung beteiligten Reaktivgruppen typischerweise verwendet werden, bevorzugt bei einer Temperatur im Bereich von 20 bis 160°C, insbesondere 40 bis 140 °C. Die Umsetzung kann unter Einsatz eines Lösemittels oder bevorzugt lösemittelfrei erfolgen. Gegebenenfalls können Hilfsstoffe wie beispielsweise Katalysatoren, Initiatoren oder Stabilisatoren mitverwendet werden. Bevorzugt werden bei der Umsetzung keine Lösemittel und Hilfsstoffe eingesetzt.

Das Amidin der Formel HZ wird bevorzugt ungefähr stöchiometrisch oder leicht überstöchiometrisch in Bezug auf die Reaktivgruppen der funktionellen Verbindung eingesetzt. Die Umsetzung wird dabei bevorzugt so geführt, dass sämtliche Reaktivgruppen der funktionellen Verbindung umgesetzt werden.

Das Reaktionsprodukt aus dieser Umsetzung wird bevorzugt ohne Aufarbeitung oder Reinigung als Katalysator für die Vernetzung einer härtbaren Zusammensetzung verwendet. Das Reaktionsprodukt kann dabei Anteile von Nebenprodukten oder nicht umgesetzten Ausgangsstoffen enthalten.

Ein weiterer Gegenstand der Erfindung ist somit ein Verfahren zur Herstellung des Amidins der Formel (I), wobei mindestens ein Amidin der Formel HZ mit mindestens einer funktionellen Verbindung der Formel 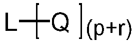 umgesetzt wird, wie vorgängig beschrieben.

Weist die funktionelle Verbindung der Formel 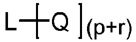 mehr als eine Reaktivgruppe auf, sind diese bevorzugt gleich, wie beispielsweise in Diglycidylen.

Als funktionelle Verbindung der Formel 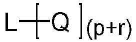 geeignet sind insbesondere im Handel erhältliche Substanzen.

Als funktionelle Verbindung der Formel 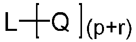 bevorzugt sind
- Epoxysilane wie insbesondere 3-Glycidoxypropyltrimethoxysilan, 3-Glycidoxypropyltriethoxysilan, 3-Glycidoxypropyldimethoxymethylsilan oder 3-Glycidoxypropyldiethoxymethylsilan.

Besonders bevorzugt ist die funktionelle Verbindung der Formel 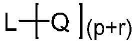 ausgewählt aus der Gruppe bestehend aus 3-Glycidoxypropyltrimethoxysilan und 3-Glycidoxypropyltriethoxysilan.

Das Amidin der Formel (I) enthält eine Silangruppe und weist insbesondere die Formel (I') auf. Dabei ist funktionelle Verbindung der Formel 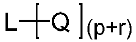 insbesondere ausgewählt aus der Gruppe bestehend aus 3-Glycidoxypropyltrimethoxysilan und 3-Glycidoxypropyltriethoxysilan.

Das Amidin der Formel HZ weist insbesondere die Formel 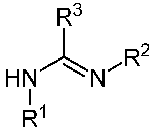 auf, wobei R¹, R² und R³ die bereits genannten Bedeutungen aufweisen.

Als Amidin der Formel HZ bevorzugt ist ein cyclisches Amidin der Formel 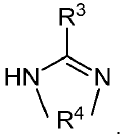 Ein solches cyclisches Amidin ist besonders einfach herstellbar.

Das cyclische Amidin der Formel HZ ist bevorzugt ausgewählt aus der Gruppe bestehend aus Imidazolin, 2-Methylimidazolin, 4(5)-Methylimidazolin, 2,4(5)-Dimethylimidazolin, 4,4(5,5)-Dimethylimidazolin, 2,4,4(2,5,5)-Trimethylimidazolin, 1,4,5,6-Tetrahydropyrimidin, 2-Methyl-1,4,5,6-tetrahydropyrimidin, 5,5-Dimethyl-1,4,5,6-tetrahydropyrimidin, 2,5,5-Trimethyl-1,4,5,6-tetrahydropyrimidin, 4(6)-Methyl-1,4,5,6-tetrahydropyrimidin, 2,4(2,6)-Dimethyl-1,4,5,6-tetrahydropyrimidin, 4(6)-Ethyl-1,4,5,6-tetrahydropyrimidin und 4(6)-Ethyl-2-methyl-1,4,5,6-tetrahydropyrimidin.

Davon bevorzugt ist Imidazolin, 2-Methylimidazolin, 1,4,5,6-Tetrahydropyrimidin oder 2-Methyl-1,4,5,6-tetrahydropyrimidin.

Am meisten bevorzugt ist 2-Methyl-1,4,5,6-tetrahydropyrimidin.

Insbesondere bevorzugt ist ein Amidin der Formel (I), welches erhalten wurde aus der Umsetzung von 2-Methyl-1 ,4,5,6-tetrahydropyrimidin mit einer funktionelle Verbindung ausgewählt aus der Gruppe bestehend aus 3-Glycidoxypropyltrimethoxysilan und 3-Glycidoxypropyltriethoxysilan.

Ganz besonders bevorzugt ist weiterhin ein Amidin der Formel (I), welches eine Silangruppe enthält und die Formel (I') aufweist, und erhalten wurde aus der Umsetzung von 2-Methyl-1,4,5,6-tetrahydropyrimidin mit einer funktionellen Verbindung ausgewählt aus der Gruppe bestehend aus 3-Glycidoxypropyltrimethoxysilan und 3-Glycidoxypropyltriethoxysilan.

Ein cyclisches Amidin der Formel 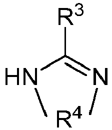 wird seinerseits insbesondere erhalten aus der Umsetzung von mindestens einem Diamin der Formel NH₂-R⁴-NH₂ mit mindestens einem Reagens zur Einführung von Amidin-Gruppen.

Das Reaktionsprodukt aus dieser Umsetzung kann ohne weitere Aufarbeitung zur Herstellung eines Amidins der Formel (I) verwendet werden.

Ein geeignetes Diamin der Formel NH₂-R⁴-NH₂ ist insbesondere Ethylendiamin, 1,2-Propandiamin, 1,3-Propandiamin, 2-Methyl-1,2-propandiamin, 2,2-Dimethyl-1,3-propandiamin, 1,3-Butandiamin, 1,4-Butandiamin, 1,3-Pentandiamin (DAMP), 1,2-Diaminocyclohexan, 1,3-Diaminocyclohexan oder 2(4)-Methyl-1,3-diaminocyclohexan. Bevorzugt ist Ethylendiamin, 1,2-Propandiamin, 1,3-Propandiamin, 2-Methyl-1,2-propandiamin, 2,2-Dimethyl-1,3-propandiamin, 1,3-Butandiamin oder 1,3-Pentandiamin (DAMP). Besonders bevorzugt ist Ethylendiamin oder 1,3-Propandiamin.

Am meisten bevorzugt ist 1,3-Propandiamin.

Das Reagens zur Einführung von Amidin-Gruppen ist bevorzugt ausgewählt aus der Gruppe bestehend aus Orthoestern, 1,3-Ketoestern, 1,3-Ketoamiden, Nitrilen, Imidsäureestern, Imidsäurechloriden, Amiden und Lactamen.

Davon bevorzugt sind Orthoester, 1,3-Ketoester oder Nitrile.

Als Orthoester bevorzugt sind Orthoester der Formel R³-C(OR⁶)₃, wobei R⁶ für einen Alkylrest mit 1 bis 4 C-Atomen steht und R³ die bereits genannten Bedeutungen aufweist, insbesondere ein Orthoformiat, Orthoacetat, Orthopropionat, Orthobutyrat oder Orthovalerat, besonders bevorzugt Trimethylorthoformiat, Triethylorthoformiat, Trimethylorthoacetat oder Triethylorthoacetat.

Als 1,3-Ketoester bevorzugt sind 1,3-Ketoester der Formel R³-C(O)CH₂C(O)OR⁶, wobei R⁶ und R³ die bereits genannten Bedeutungen aufweisen, insbesondere Methylacetoacetat, Ethylacetoacetat, Isopropylacetoacetat oder tert.Butylacetoacetat, besonders bevorzugt Ethylacetoacetat.

Als Nitril bevorzugt sind Nitrile der Formel R³-CN, wobei R³ die bereits genannten Bedeutungen aufweist, insbesondere Acetonitril, Propionitril, Butyronitril, Isobutyronitril, Valeronitril oder Capronitril, besonders bevorzugt Acetonitril.

Das Reagens zur Einführung von Amidin-Gruppen ist besonders bevorzugt ausgewählt aus der Gruppe bestehend aus Trimethylorthoformiat, Triethylorthoformiat, Trimethylorthoacetat, Triethylorthoacetat, Methylacetoacetat, Ethylacetoacetat, Isopropylacetoacetat, tert.Butylacetoacetat und Acetonitril. Mit diesen Reagentien werden auf besonders einfache Weise cyclische Amidine der Formel HZ erhalten, welche Amidine der Formel (I) mit besonders hoher katalytischer Aktivität ermöglichen.

Die Umsetzung wird bevorzugt bei erhöhter Temperatur, gegebenenfalls unter erhöhtem Druck und gegebenenfalls in Gegenwart eines Katalysators, durchgeführt, wobei aus dem Reagens freigesetzte Abspalter wie Alkohole, Ester oder Amine während oder nach der Umsetzung bevorzugt entfernt werden, insbesondere mittels Destillation, gegebenenfalls unter Vakuum.

Bevorzugt wird das Verhältnis zwischen dem Diamin und dem Reagens so gewählt, dass das Reagens bei der Umsetzung vollständig umgesetzt wird. Besonders bevorzugt werden das Diamin und das Reagens im Molverhältnis von ungefähr 1:1 bis 1.5:1, insbesondere 1:1 bis 1.2:1, eingesetzt.

Wird ein Orthoester eingesetzt, so erfolgt die Umsetzung bevorzugt bei einer Temperatur von 40 bis 160°C, insbesondere 60 bis 140°C, wobei der freigesetzte Alkohol bevorzugt destillativ entfernt wird. Gegebenenfalls wird dabei ein Katalysator eingesetzt, insbesondere eine Säure.

Wird ein 1,3-Ketoester eingesetzt, so erfolgt die Umsetzung bevorzugt bei einer Temperatur von 20 bis 100°C, insbesondere 40 bis 80°C, wobei der freigesetzte Ester bevorzugt destillativ entfernt wird. Bevorzugt wird dabei ein Katalysator eingesetzt, insbesondere eine Säure, bevorzugt eine Sulfonsäure.

Wird ein Nitril eingesetzt, so erfolgt die Umsetzung bevorzugt bei einer Temperatur von 60 bis 180°C, insbesondere 80 bis 160°C, gegebenenfalls unter erhöhtem Druck, wobei der freigesetzte Ammoniak bevorzugt destillativ entfernt wird. Bevorzugt wird dabei ein Katalysator eingesetzt, insbesondere eine Lewissäure, bevorzugt Bortrifluorid-Etherat, Lithiumperchlorat, Zinkchlorid, Zink(III)-trifluormethansulfonat oder Lanthan(III)trifluormethansulfonat.

In einem bevorzugten Amidin der Formel (I) stehen p für 1, r für 0, Q' für 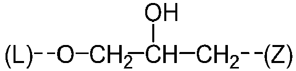 und Z für eine cyclische Amidingruppe. Ein solches Amidin weist insbesondere die Formel (la) auf.

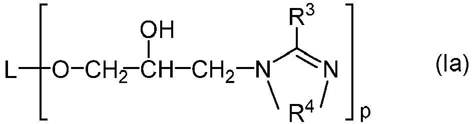

L steht dabei bevorzugt für 3-Glycidoxypropyltrimethoxysilan und 3-Glycidoxypropyltriethoxysilan jeweils nach Entfernung der Glycidoxygruppen. Besonders bevorzugt stehen p für 1 und L für Triethoxysilylpropyl oder Trimethoxysilylpropyl. Ein solches Amidin ist besonders geeignet als Katalysator für Silangruppen-haltige Zusammensetzungen, wo es bei der Aushärtung über die Silangruppen kovalent gebunden werden kann.

Ein weiterer Gegenstand der Erfindung ist die Verwendung eines Amidins der Formel (I) wie vorgängig beschrieben als Katalysator für die Vernetzung einer härtbaren Zusammensetzung. Dabei beschleunigt es die Vernetzung bzw. Aushärtung der Zusammensetzung.

Als härtbare Zusammensetzung geeignet ist insbesondere
- eine Epoxidharz-Zusammensetzung, insbesondere ein heisshärtendes, über Dicyandiamid oder Carbonsäuren oder Carbonsäureanhydride vernetzendes System, wie es beispielsweise in Klebstoffen, Beschichtungen oder Giessharzen eingesetzt wird; oder
- eine Polyurethan-Zusammensetzung, insbesondere ein zweikomponentiges, durch Reaktion von Polyolen mit Isocyanaten vernetzendes System, wie es beispielsweise für Klebstoffe, Beläge, Vergussmassen, Dichtfugen, Formkörper oder Blockschäume eingesetzt wird, oder ein einkomponentiges System mit blockierten Isocyanatgruppen oder blockierten Aminogruppen, wie es beispielsweise in Pulverlacken, Coil Coatings, Elektrotauchlacken oder Flüssiglacken eingesetzt wird; oder
- eine Epoxidharz-Polyurethan-Zusammensetzung; oder
- eine Cyanatesterharz-Zusammensetzung; oder
- eine Silangruppen-haltige Zusammensetzung.

Bevorzugt ist die härtbare Zusammensetzung eine Polyurethan-Zusammensetzung oder eine Silangruppen-haltige Zusammensetzung. In einer solchen Zusammensetzung ermöglicht das Amidin der Formel (I) eine gute Lagerstabilität und eine schnelle Aushärtung.

Besonders vorteilhaft ist die Verwendung zur Vernetzung einer Silangruppen-haltigen Zusammensetzung, insbesondere einer Zusammensetzung auf Basis von Silangruppen-haltigen Polymeren. Zusammensetzungen auf Basis von Silangruppen-haltigen Polymeren härten auch bei relativ geringer Katalysator-Konzentration rasch aus und neigen nicht zu migrationsbedingten Fehlern wie Separation, Ausschwitzen oder Substratverschmutzung.

Besonders bevorzugt ist die Verwendung des Amidins der Formel (I) als Katalysator für die Vernetzung einer Zusammensetzung auf Basis von Silangruppen-haltigen Polymeren ausgewählt aus der Gruppe bestehend aus Polyorganosiloxanen mit endständigen Silangruppen und Silangruppen-haltigen organischen Polymeren.

Ein Polyorganosiloxan mit endständigen Silangruppen weist den Vorteil auf, dass es im ausgehärteten Zustand besonders wasser- und lichtbeständig ist und besonders weichelastische Eigenschaften ermöglicht.

Ein Silangruppen-haltiges organisches Polymer weist den Vorteil auf, dass es besonders gute Haftungseigenschaften auf einer Vielzahl von Untergründen aufweist und besonders kostengünstig ist.

Ein weiterer Gegenstand der Erfindung ist somit eine härtbare Zusammensetzung enthaltend mindestens ein Amidin der Formel (I).

Bevorzugt stellt die härtbare Zusammensetzung einen Klebstoff oder einen Dichtstoff oder eine Beschichtung dar.

Bevorzugt enthält die härtbare Zusammensetzung weiterhin mindestens ein Silangruppen-haltiges Polymer.

Eine solche Zusammensetzung verfügt typischerweise über eine gute Lagerfähigkeit ohne Separationsneigung, erlaubt aufgrund der geringen Toxizität und geringen Flüchtigkeit des Amidins der Formel (I) eine niedrige Gefahreneinstufung und ermöglicht emissions- und geruchsarme Produkte, welche rasch aushärten und dabei ein mechanisch hochwertiges und beständiges Material bilden. Besonders vorteilhaft ist dabei der Umstand, dass dieses Material kaum zu migrationsbedingten Fehlern wie Ausschwitzen oder Substratverschmutzung neigt, im Gegensatz zu Zusammensetzungen mit Katalysatoren nach dem Stand der Technik, wie beispielsweise DBU oder TMG. Zusammensetzungen enthaltend solche aus dem Stand der Technik bekannten Katalysatoren neigen zu Migrationseffekten, was sich vor der Aushärtung durch Separation und nach der Aushärtung durch klebrige und/oder schmierige Oberflächen und/oder Substratverschmutzungen äussern kann. Besonders letztere Effekte sind äusserst unerwünscht, da klebrige und schmierige Oberflächen schnell verschmutzen und schlecht überstreichbar sind, und Substratverunreinigungen zu bleibenden Verfärbungen führen können.

Das Silangruppen-haltige Polymer ist in einer bevorzugten Ausführungsform ein Polyorganosiloxan mit endständigen Silangruppen.

Ein bevorzugtes Polyorganosiloxan mit endständigen Silangruppen weist die Formel (IV) auf, 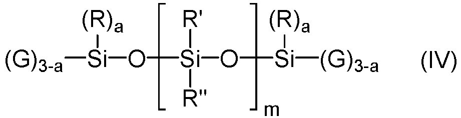 wobei
R, R' und R" unabhängig voneinander jeweils für einen einwertigen Kohlenwasserstoff-Rest mit 1 bis 12 C-Atomen stehen;
G für einen Hydroxyl-Rest oder für einen Alkoxy-, Acetoxy-, Ketoximato-, Amido- oder Enoxy-Rest mit 1 bis 13 C-Atomen steht;
a für 0, 1 oder 2 steht; und
m für eine ganze Zahl im Bereich von 50 bis etwa 2'500 steht.

R steht bevorzugt für Methyl, Vinyl oder Phenyl.

R' und R" stehen bevorzugt unabhängig voneinander jeweils für einen Alkylrest mit 1 bis 5, bevorzugt 1 bis 3 C-Atomen, insbesondere für Methyl.

G steht bevorzugt für einen Hydroxyl-Rest oder für einen Alkoxy- oder Ketoximato-Rest mit 1 bis 6 C-Atomen, insbesondere für einen Hydroxyl-, Methoxy-, Ethoxy-, Methylethylketoximato- oder Methylisobutylketoximato-Rest. Besonders bevorzugt steht G für einen Hydroxylrest.

a steht bevorzugt für 0 oder 1, insbesondere für 0.

Weiterhin ist m bevorzugt so gewählt, dass das Polyorganosiloxan der Formel (IV) bei Raumtemperatur eine Viskosität im Bereich von 100 bis 500'000 mPa·s, insbesondere von 1000 bis 100'000 mPa s, aufweist.

Polyorganosiloxane der Formel (IV) sind gut handhabbar und vernetzen mit Feuchtigkeit und/oder Silanvernetzern zu festen Silicon-Polymeren mit elastischen Eigenschaften.

Geeignete kommerziell erhältliche Polyorganosiloxane der Formel (IV) sind beispielsweise erhältlich von Wacker, Momentive Performance Material, GE Advanced Materials, Dow Corning, Bayer oder Shin Etsu.

Bevorzugt enthält die Zusammensetzung zusätzlich zum Polyorganosiloxan mit endständigen Silangruppen einen Silanvernetzer, insbesondere ein Silan der Formel (V), 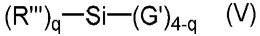 wobei
R‴ für einen einwertigen Kohlenwasserstoff-Rest mit 1 bis 12 C-Atomen steht, G' für einen Hydroxyl-Rest oder für einen Alkoxy-, Acetoxy-, Ketoximato-, Amido- oder Enoxy-Rest mit 1 bis 13 C-Atomen steht; und
q für einen Wert von 0, 1 oder 2, insbesondere für 0 oder 1, steht.

Besonders geeignete Silane der Formel (V) sind Methyltrimethoxysilan, Ethyltrimethoxysilan, Propytrimethoxysilan, Vinyltrimethoxysilan, Methyltriethoxysilan, Vinyltriethoxysilan, Phenyltriethoxysilan, Tetramethoxysilan, Tetraethoxysilan, Methyltris(methylethylketoximo)silan, Vinyltris(methylethylketoximo)silan und Methyltris(isobutylketoximo)silan.

Das Silangruppen-haltige Polymer ist in einer weiteren bevorzugten Ausführungsform ein Silangruppen-haltiges organisches Polymer, insbesondere ein Polyolefin, Polyester, Polyamid, Poly(meth)acrylat oder Polyether oder eine Mischform dieser Polymere, welches jeweils eine oder bevorzugt mehrere Silangruppen trägt. Die Silangruppen können seitlich in der Kette oder endständig stehen und sind über ein C-Atom an das organische Polymer gebunden. Besonders bevorzugt ist das Silangruppen-haltige organische Polymer ein Silangruppen-haltiges Polyolefin oder ein Silangruppen-haltiger Polyester oder ein Silangruppen-haltiges Poly(meth)acrylat oder ein Silangruppen-haltiger Polyether oder eine Mischform dieser Polymere.

Am meisten bevorzugt ist das Silangruppen-haltige organische Polymer ein Silangruppen-haltiger Polyether.

Das Silangruppen-haltige organische Polymer weist als Silangruppen bevorzugt Alkoxysilangruppen auf, insbesondere Alkoxysilangruppen der Formel (VI), 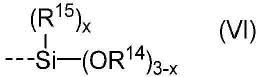 wobei
R¹⁴ für einen linearen oder verzweigten, einwertigen Kohlenwasserstoffrest mit 1 bis 5 C-Atomen, insbesondere für Methyl oder für Ethyl oder für Isopropyl, steht;
R¹⁵ für einen linearen oder verzweigten, einwertigen Kohlenwasserstoffrest mit 1 bis 8 C-Atomen, insbesondere für Methyl oder für Ethyl, steht; und x für einen Wert von 0 oder 1 oder 2, bevorzugt für 0 oder 1, insbesondere für 0, steht.

Besonders bevorzugt steht R¹⁴ für Methyl oder für Ethyl.

Besonders bevorzugt sind Trimethoxysilangruppen, Dimethoxymethylsilangruppen oder Triethoxysilangruppen.

Dabei weisen Methoxysilangruppen den Vorteil auf, dass sie besonders reaktiv sind, und Ethoxysilangruppen weisen den Vorteil auf, dass sie toxikologisch vorteilhaft und besonders lagerstabil sind.

Das Silangruppen-haltige organische Polymer weist im Mittel bevorzugt 1.3 bis 4, insbesondere 1.5 bis 3, besonders bevorzugt 1.7 bis 2.8, Silangruppen pro Molekül auf. Die Silangruppen sind bevorzugt endständig.

Das Silangruppen-haltige organische Polymer weist bevorzugt ein mittleres Molekulargewicht im Bereich von 1'000 bis 30'000 g/mol, insbesondere von 2'000 bis 20'000 g/mol, auf. Das Silangruppen-haltige organische Polymer weist bevorzugt ein Silan-Equivalentgewicht von 300 bis 25'000 g/Eq, insbesondere von 500 bis 15'000 g/Eq, auf.

Das Silangruppen-haltige organische Polymer kann bei Raumtemperatur fest oder flüssig vorliegen. Bevorzugt ist es bei Raumtemperatur flüssig.

Meist bevorzugt handelt es sich beim Silangruppen-haltigen organischen Polymer um einen bei Raumtemperatur flüssigen Silangruppen-haltigen Polyether, wobei die Silangruppen insbesondere Dialkoxysilangruppen und/oder Trialkoxysilangruppen, besonders bevorzugt Trimethoxysilangruppen oder Triethoxysilangruppen, sind.

Verfahren zur Herstellung von Silangruppen-haltigen Polyethern sind dem Fachmann bekannt.

In einem bevorzugten Verfahren sind Silangruppen-haltige Polyether erhältlich aus der Umsetzung von Allylgruppen-haltigen Polyethern mit Hydrosilanen, gegebenenfalls unter Kettenverlängerung mit beispielsweise Diisocyanaten.

In einem weiteren bevorzugten Verfahren sind Silangruppen-haltige Polyether erhältlich aus der Copolymerisation von Alkylenoxiden und Epoxysilanen, gegebenenfalls unter Kettenverlängerung mit beispielsweise Diisocyanaten.

In einem weiteren bevorzugten Verfahren sind Silangruppen-haltige Polyether erhältlich aus der Umsetzung von Polyetherpolyolen mit Isocyanatosilanen, gegebenenfalls unter Kettenverlängerung mit Diisocyanaten.

In einem weiteren bevorzugten Verfahren sind Silangruppen-haltige Polyether erhältlich aus der Umsetzung von Isocyanatgruppen-haltigen Polyethern, insbesondere NCO-terminierten Urethan-Polyethern aus der Umsetzung von Polyetherpolyolen mit einer überstöchiometischen Menge an Polyisocyanaten, mit Aminosilanen, Hydroxysilanen oder Mercaptosilanen. Silangruppen-haltige Polyether aus diesem Verfahren sind besonders bevorzugt. Dieses Verfahren ermöglicht den Einsatz einer Vielzahl von kommerziell gut verfügbaren, kostengünstigen Ausgangsmaterialien, womit unterschiedliche Polymereigenschaften erhalten werden können, beispielsweise eine hohe Dehnbarkeit, eine hohe Festigkeit, ein niedriges Elastizitätsmodul, ein tiefer Glasübergangspunkt oder eine hohe Witterungsbeständigkeit.

Besonders bevorzugt ist der Silangruppen-haltige Polyether erhältlich aus der Umsetzung von NCO-terminierten Urethan-Polyethern mit Aminosilanen oder Hydroxysilanen. Geeignete NCO-terminierte Urethan-Polyether sind erhältlich aus der Umsetzung von Polyetherpolyolen, insbesondere Polyoxyalkylendiolen oder Polyoxyalkylentriolen, bevorzugt Polyoxypropylendiolen oder Polyoxypropylentriolen, mit einer überstöchiometrischen Menge an Polyisocyanaten, insbesondere Diisocyanaten.

Bevorzugt wird die Umsetzung zwischen dem Polyisocyanat und dem Polyetherpolyol unter Feuchtigkeitsausschluss bei einer Temperatur von 50 °C bis 160 °C durchgeführt, gegebenenfalls in Anwesenheit geeigneter Katalysatoren, wobei das Polyisocyanat so dosiert ist, dass dessen Isocyanatgruppen im Verhältnis zu den Hydroxylgruppen des Polyols im stöchiometrischen Überschuss vorhanden sind. Insbesondere wird der Überschuss an Polyisocyanat so gewählt, dass im resultierenden Urethan-Polyether nach der Umsetzung aller Hydroxylgruppen ein Gehalt an freien Isocyanatgruppen von 0.1 bis 5 Gewichts-%, bevorzugt 0.2 bis 4 Gewichts-%, besonders bevorzugt 0.3 bis 3 Gewichts-%, bezogen auf das gesamte Polymer, verbleibt.

Bevorzugte Diisocyanate sind ausgewählt aus der Gruppe bestehend aus 1,6-Hexamethylendiisocyanat (HDI), 1-Isocyanato-3,3,5-trimethyl-5-isocyanatomethyl-cyclohexan (Isophorondiisocyanat oder IPDI), 2,4- und 2,6-Toluylendiisocyanat und beliebige Gemische dieser Isomeren (TDI) und 4,4'-, 2,4'- und 2,2'-Diphenylmethandiisocyanat und beliebige Gemische dieser Isomeren (MDI).

Besonders bevorzugt sind IPDI oder TDI. Meist bevorzugt ist IPDI. Damit werden Silangruppen-haltige Polyether mit besonders guter Lichtechtheit erhalten.

Speziell geeignet als Polyetherpolyole sind Polyoxyalkylendiole oder Polyoxyalkylentriole mit einem Ungesättigtheitsgrad tiefer als 0.02 mEq/g, insbesondere tiefer als 0.01 mEq/g, und einem mittleren Molekulargewicht im Bereich von 400 bis 25'000 g/mol, insbesondere 1000 bis 20'000 g/mol.

Neben Polyetherpolyolen können anteilig auch andere Polyole eingesetzt werden, insbesondere Polyacrylatpolyole, sowie niedrigmolekulare Diole oder Triole.

Geeignete Aminosilane für die Umsetzung mit einem NCO-terminierten Urethan-Polyether sind primäre und sekundäre Aminosilane. Bevorzugt sind 3-Aminopropyltrimethoxysilan, 3-Aminopropyldimethoxymethylsilan, 4-Aminobutyltrimethoxysilan, 4-Amino-3-methylbutyltrimethoxysilan, 4-Amino-3,3-di-methylbutyltrimethoxysilan, N-Butyl-3-aminopropyltrimethoxysilan, N-Phenyl-3-aminopropyltrimethoxysilan, Addukte aus primären Aminosilanen wie 3-Aminopropyltrimethoxysilan, 3-Aminopropyldimethoxymethylsilan oder N-(2-Aminoethyl)-3-aminopropyltrimethoxysilan und Michael-Akzeptoren wie Acrylnitril, (Meth)acrylsäureestern, (Meth)acrylsäureamiden, Maleinsäure- oder Fumarsäurediestern, Citraconsäurediestern oder Itaconsäurediestern, insbesondere N-(3-Trimethoxysilylpropyl)aminobernsteinsäuredimethyl- oder -diethylester. Ebenfalls geeignet sind Analoga der genannten Aminosilane mit Ethoxy- oder Isopropoxygruppen anstelle der Methoxygruppen am Silicium.

Geeignete Hydroxysilane für die Umsetzung mit einem NCO-terminierten Urethan-Polyether sind insbesondere erhältlich aus der Addition von Aminosilanen an Lactone oder an cyclische Carbonate oder an Lactide.

Dafür geeignete Aminosilane sind insbesondere 3-Aminopropyltrimethoxysilan, 3-Aminopropyltriethoxysilan, 4-Aminobutyltrimethoxysilan, 4-Aminobutyltriethoxysilan, 4-Amino-3-methylbutyltrimethoxysilan, 4-Amino-3-methylbutyltriethoxy-silan, 4-Amino-3,3-dimethylbutyltrimethoxysilan, 4-Amino-3,3-dimethylbutyltri-ethoxysilan, 2-Aminoethyltrimethoxysilan oder 2-Aminoethyltriethoxysilan. Besonders bevorzugt sind 3-Aminopropyltrimethoxysilan, 3-Aminopropyltriethoxysilan, 4-Amino-3,3-dimethylbutyltrimethoxysilan oder 4-Amino-3,3-dimethyl-butyltriethoxysilan.

Als Lactone geeignet sind insbesondere γ-Valerolacton, γ-Octalacton, δ-Decalacton, und ε-Decalacton, insbesondere γ-Valerolacton.

Als cyclische Carbonate eignen sich insbesondere 4,5-Dimethyl-1,3-dioxolan-2-on, 4,4-Dimethyl-1,3-dioxolan-2-on, 4-Ethyl-1,3-dioxolan-2-on, 4-Methyl-1,3-dioxolan-2-on oder 4-(Phenoxymethyl)-1,3-dioxolan-2-on.

Als Lactide eignen sich insbesondere 1,4-Dioxan-2,5-dion (Lactid aus 2-Hydroxyessigsäure, auch "Glycolid" genannt), 3,6-Dimethyl-1,4-dioxan-2,5-dion (Lactid aus Milchsäure, auch "Lactid" genannt) und 3,6-Diphenyl-1,4-dioxan-2,5-dion (Lactid aus Mandelsäure).

Bevorzugte Hydroxysilane, welche auf diese Weise erhalten werden, sind N-(3-Triethoxysilylpropyl)-2-hydroxypropanamid, N-(3-Trimethoxysilylpropyl)-2-hydroxypropanamid, N-(3-Triethoxysilylpropyl)-4-hydroxypentanamid, N-(3-Triethoxysilylpropyl)-4-hydroxyoctanamid, N-(3-Triethoxysilylpropyl)-5-hydroxydecanamid und N-(3-Triethoxysilylpropyl)-2-hydroxypropylcarbamat.

Weiterhin sind geeignete Hydroxysilane auch erhältlich aus der Addition von Aminosilanen an Epoxide oder aus der Addition von Aminen an Epoxysilane.

Bevorzugte Hydroxysilane, welche auf diese Weise erhalten werden, sind 2-Morpholino-4(5)-(2-trimethoxysilylethyl)cyclohexan-1-ol, 2-Morpholino-4(5)-(2-triethoxysilylethyl)cyclohexan-1-ol oder 1-Morpholino-3-(3-(triethoxysilyl)propoxy)propan-2-ol.

Als Silangruppen-haltige Polyether sind auch kommerziell erhältliche Produkte geeignet, insbesondere die Folgenden: MS Polymer^{™} (von Kaneka Corp.; insbesondere die Typen S203H, S303H, S227, S810, MA903 und S943); MS Polymer^{™} bzw. Silyl^{™} (von Kaneka Corp.; insbesondere die Typen SAT010, SAT030, SAT200, SAX350, SAX400, SAX725, MAX450, MAX951); Excestar^{®} (von Asahi Glass Co. Ltd.; insbesondere die Typen S2410, S2420, S3430, S3630); SPUR+* (von Momentive Performance Materials; insbesondere die Typen 1010LM, 1015LM, 1050MM); Vorasil^{™} (von Dow Chemical Co.; insbesondere die Typen 602 und 604); Desmoseal^{®} (von Bayer MaterialScience AG; insbesondere die Typen S XP 2458, S XP 2636, S XP 2749, S XP 2774 und S XP 2821), TEGOPAC^{®} (von Evonik Industries AG; insbesondere die Typen Seal 100, Bond 150, Bond 250), Polymer ST (von Hanse Chemie AG/Evonik Industries AG, insbesondere die Typen 47, 48, 61, 61LV, 77, 80, 81); Geniosil^{®} STP (von Wacker Chemie AG; insbesondere die Typen E10, E15, E30, E35).

Besonders bevorzugte Silangruppen-haltige organische Polymere weisen Endgruppen der Formel (VII) auf, 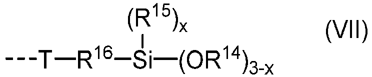 wobei
R¹⁶ für einen linearen oder verzweigten, zweiwertigen Kohlenwasserstoffrest mit 1 bis 12 C-Atomen, welcher gegebenenfalls cyclische und/oder aromatische Anteile und gegebenenfalls ein oder mehrere Heteroatome, insbesondere ein oder mehrere Stickstoffatome, aufweist, steht;
T für einen zweiwertigen Rest ausgewählt aus -O-, -S-, -N(R¹⁷)-, -O-CO-N(R¹⁷)-, -N(R¹⁷)-CO-O- und -N(R¹⁷)-CO-N(R¹⁷)- steht,
   wobei R¹⁷ für einen Wasserstoff-Rest oder für einen linearen oder verzweigten Kohlenwasserstoff-Rest mit 1 bis 20 C-Atomen, welcher gegebenenfalls cyclische Anteile aufweist, und welcher gegebenenfalls eine Alkoxysilan-, Ether- oder Carbonsäureester-Gruppe aufweist, steht; und
R¹⁴, R¹⁵ und x die bereits genannten Bedeutungen aufweisen.

Bevorzugt steht R¹⁶ für 1,3-Propylen oder für 1,4-Butylen, wobei Butylen mit einer oder zwei Methylgruppen substituiert sein kann.

Besonders bevorzugt steht R¹⁶ für 1,3-Propylen.

Bevorzugt ist das Amidin der Formel (I) in der härtbaren Zusammensetzung in einer solchen Menge vorhanden, dass die Konzentration an Amidin-Gruppen bezogen auf die Menge des vernetzbaren Polymers im Bereich von 0.1 bis 50 mmol/100 g Polymer, bevorzugt 0.2 bis 50 mmol/100 g Polymer, insbesondere 0.5 bis 20 mmol/100 g, liegt.

Eine solche Zusammensetzung weist eine gute Lagerfähigkeit und eine schnelle Aushärtung auf.

Zusätzlich zum Amidin der Formel (I) kann die Zusammensetzung weitere Katalysatoren, insbesondere für die Vernetzung von Silangruppen, enthalten. Als weitere Katalysatoren geeignet sind insbesondere Metall-Verbindungen und/oder basische Stickstoff- oder Phosphorverbindungen.

Geeignete Metall-Verbindungen sind insbesondere Verbindungen von Zinn, Titan, Zirkonium, Aluminium oder Zink, insbesondere Diorganozinn(IV)-Verbindungen wie insbesondere Dibutylzinn(IV)-diacetat, Dibutylzinn(IV)-dilaurat, Dibutylzinn(IV)-dineodecanoat oder Dibutylzinn(IV)-bis(acetylacetonat) und Dioctylzinn(IV)-dilaurat, sowie Titan(IV)- oder Zirkonium(IV)- oder Aluminium(III)- oder Zink(II)-Komplexe mit insbesondere Alkoxy-, Carboxylat-, 1,3-Diketonat-, 1,3-Ketoesterat- oder 1,3-Ketoamidat-Liganden.

Geeignete basische Stickstoff- oder Phosphorverbindungen sind insbesondere Imidazole, Pyridine, Phosphazen-Basen oder bevorzugt Amine, Hexahydrotriazine, Biguanide, Guanidine oder weitere Amidine.

Geeignete Amine sind insbesondere Alkyl-, Cycloalkyl- oder Aralkylamine wie Triethylamin, Triisopropylamin, 1-Butylamin, 2-Butylamin, tert.Butylamin, 3-Methyl-1-butylamin, 3-Methyl-2-butylamin, Dibutylamin, Tributylamin, Hexylamin, Dihexylamin, Cyclohexylamin, Dicyclohexylamin, Dimethylcyclohexylamin, Benzylamin, Dibenzylamin, Dimethylbenzylamin, Octylamin, 2-Ethylhexylamin, Di-(2-ethylhexyl)amin, Laurylamin, N,N-Dimethyl-Iaurylamin, Stearylamin, N,N-Dimethyl-stearylamin; von natürlichen Fettsäuregemischen abgeleitete Fettamine, wie insbesondere Cocoalkylamin, N,N-Dimethyl-cocoalkylamin, C₁₆₋₂₂-Alkylamin, N,N-Dimethyl-C₁₆₋₂₂-alkylamin, Soyaalkylamin, N,N-Dimethyl-soyaalkylamin, Oleylamin, N,N-Dimethyl-oleylamin, Talgalkylamin oder N,N-Dimethyl-talgalkylamin, erhältlich beispielsweise unter den Handelsnamen Armeen^{®} (von Akzo Nobel) oder Rofamin^{®} (von Ecogreen Oleochemicals); aliphatische, cycloaliphatische oder araliphatische Diamine wie Ethylendiamin, Butandiamin, Hexamethylendiamin, Dodecandiamin, Neopentandiamin, 2-Methyl-pentamethylendiamin (MPMD), 2,2(4),4-Trimethylhexamethylendiamin (TMD), Isophorondiamin (IPD), 2,5(2,6)-Bis-(aminomethyl)-bicy-clo[2.2.1]heptan (NBDA), 1,3-Xylylendiamin (MXDA), N,N'-Di(tert.butyl)ethylendiamin, N,N,N',N'-Tetramethyl-ethylendiamin, N,N,N',N'-Tetramethyl-propylendiamin, N,N,N',N'-Tetramethyl-hexamethylendiamin, 3-Dimethylaminopropylamin, 3-(Methylamino)propylamin, 3-(Cyclohexylamino)propylamin, Piperazin, N-Methylpiperazin, N,N'-Dimethylpiperazin, 1,4-Diazabicyclo[2.2.2]octan, Fettpolyamine wie N-Cocoalkyl-1,3-propandiamin, N-Oleyl-1,3-propandiamin, N-Soyaalkyl-1,3-propandiamin, N-Talgalkyl-1,3-propandiamin oder N-(C₁₆₋₂₂-Alkyl)-1,3-propandiamin, erhältlich beispielsweise unter dem Handelsnamen Duomeen^{®} (von Akzo Nobel); Polyalkylenamine wie Diethylentriamin, Dipropylentriamin, Triethylentetramin (TETA), Tetraethylenpentamin (TEPA), Pentamethylenhexamin (PEHA), 3-(2-Aminoethyl)aminopropylamin, N,N'-Bis(3-aminopropyl)ethylendiamin, N-(3-Aminopropyl)-N-methylpropandiamin, Bis(3-dimethylaminopropyl)amin, N-(3-Dimethylaminopropyl)-1,3-propylendiamin, N-(2-Aminoethyl)piperazin (N-AEP), N-(2-Aminopropyl)piperazin, N,N'-Di-(2-aminoethyl)piperazin, 1-Methyl-4-(2-dimethylaminoethyl)piperazin, N,N,N',N",N"-Pentamethyldiethylentriamin, N,N,N',N",N"-Pentamethyldipropylentriamin, Polyethylenimine, erhältlich beispielsweise unter den Handelsnamen Lupasol^{®} (von BASF) und Epomin^{®} (von Nippon Shokubai); Etheramine, wie insbesondere 2-Methoxyethylamin, 2-Ethoxyethylamin, 3-Methoxypropylamin, 3-Ethoxypropylamin, 3-(2-Ethylhexyloxy)propylamin, 3-(2-Methoxyethoxy)propylamin, 2(4)-Methoxyphenylethylamin, Morpholin, N-Methylmorpholin, N-Ethylmorpholin, 2-Aminoethylmorpholin, Bis(2-aminoethyl)ether, Bis(dimethylaminoethyl)-ether, Bis(dimorpholinoethyl)ether, N,N,N'-Trimethyl-N'-hydroxyethylbis(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadode-can-3,10-diamin, 4,7,10-Trioxatridecan-1,13-diamin oder 2-Aminopropyl-terminierte Glykole, wie sie beispielsweise unter dem Handelsnamen Jeffamin^{®} (von Huntsman) erhältlich sind; Aminoalkohole, wie insbesondere Ethanolamin, Isopropanolamin, Diethanolamin, Diisopopanolamin, Triethanolamin, Triisopropanolamin, N-Butylethanolamin, Diglykolamin, N,N-Diethylethanolamin, N-Methyldiethanolamin, N-Methyldiisopropylamin, N,N,N'-Trimethylaminoethyl-ethanolamin, N-(3-Dimethylaminopropyl)-N,N-diisopropanolamin, N,N-Bis(3-dimethylaminopropyl)-N-isopropanolamin, 2-(2-Dimethylaminoethoxy)ethanolamin oder Addukte aus Mono- und Polyaminen mit Epoxiden oder Diepoxiden; Phenolgruppen-haltige Amine, wie insbesondere Kondensationsprodukte aus Phenolen, Aldehyden und Aminen (sogenannte Mannich-Basen und Phenalkamine) wie insbesondere 2-(Dimethylaminomethyl)phenol, 2,4,6-Tris(dimethylaminomethyl)phenol oder Polymere aus Phenol, Formaldehyd und N,N-Dimethyl-1,3-propandiamin sowie im Handel unter den Markennamen Cardolite^{®} (von Cardolite), Aradur^{®} (von Huntsman) und Beckopox^{®} (von Cytec) erhältliche Phenalkamine; Amidgruppen-haltige Polyamine, sogenannte Polyamidoamine, wie sie im Handel erhältlich sind, beispielsweise unter den Markennamen Versamid^{®} (von Cognis), Aradur^{®} (von Huntsman), Euretek^{®} (von Huntsman) oder Beckopox^{®} (von Cytec); oder Aminosilane, wie insbesondere 3-Aminopropyltrimethoxysilan, 3-Aminopropyldimethoxymethylsilan, N-(2-Aminoethyl)-3-aminopropyltrimethoxysilan, N-(2-Aminoethyl)-3-aminopropyl-methyldimethoxysilan, N-(2-Aminoethyl)-N'-[3-(trimethoxysilyl)propyl]ethylendiamin oder deren Analoga mit Ethoxy- anstelle der Methoxygruppen am Silicium.

Geeignete Hexahydrotriazine sind insbesondere 1,3,5-Hexahydrotriazin oder 1,3,5-Tris(3-(dimethylamino)propyl)-hexahydrotriazin.

Geeignete Biguanide sind insbesondere Biguanid, 1-Butylbiguanid, 1,1-Dimethylbiguanid, 1-Butylbiguanid, 1-Phenylbiguanid oder 1-(o-Tolyl)biguanid (OTBG).

Geeignete Guanidine sind insbesondere 1-Butylguanidin, 1,1-Dimethylguanidin, 1,3-Dimethylguanidin, 1,1,3,3-Tetramethylguanidin (TMG), 2-(3-(Tri-methoxysilyl)propyl)-1,1,3,3-tetramethylguanidin, 2-(3-(Methyldimethoxysilyl)-propyl)-1,1,3,3-tetramethylguanidin, 2-(3-(Triethoxysilyl)propyl)-1,1,3,3-tetramethylguanidin, 1,5,7-Triazabicyclo[4.4.0]dec-5-en (TBD), 7-Methyl-1,5,7-triazabicyclo[4.4.0]dec-5-en, 7-Cyclohexyl-1,5,7-triazabicyclo[4.4.0]dec-5-en, 1-Phenylguanidin, 1-(o-Tolyl)guanidin (OTG), 1,3-Diphenylguanidin, 1,3-Di(o-tolyl)guanidin oder 2-Guanidinobenzimidazol.

Geeignete weitere Amidine sind insbesondere 1,8-Diazabicyclo[5.4.0]undec-7-en (DBU), 1,5-Diazabicyclo[4.3.0]non-5-en (DBN), 6-Dibutylamino-1,8-diazabicyclo[5.4.0]undec-7-en, 6-Dibutylamino-1,8-diazabicyclo[5.4.0]undec-7-en, N,N'-Di-n-hexylacetamidin (DHA), 2-Methyl-1,4,5,6-tetrahydropyrimidin, 1,2-Dimethyl-1,4,5,6-tetrahydropyrimidin, 2,5,5-Trimethyl-1,4,5,6-tetrahydropyrimidin, N-(3-Trimethoxysilylpropyl)-4,5-dihydroimidazol oder N-(3-Triethoxysilylpropyl)-4,5-dihydroimidazol.

Weiterhin kann die Zusammensetzung als Co-Katalysator eine Säure enthalten, insbesondere eine Carbonsäure. Bevorzugt sind aliphatische Carbonsäuren wie Ameisensäure, Laurinsäure, Stearinsäure, Isostearinsäure, Ölsäure, 2-Ethyl-2,5-dimethylcapronsäure, 2-Ethylhexansäure, Neodecansäure, Fettsäuregemische aus der Verseifung von natürlichen Fetten und Ölen oder Di- und Polycarbonsäuren, insbesondere Poly(meth)acrylsäuren.

Die Zusammensetzung ist in einer bevorzugten Ausführungsform im Wesentlichen frei von Organozinn-Verbindungen. Organozinn-freie Zusammensetzungen sind hinsichtlich Gesundheitsschutz und Umweltschutz vorteilhaft. Insbesondere beträgt der Zinngehalt der härtbaren Zusammensetzung weniger als 0.1 Gew.-%, insbesondere weniger als 0.05 Gew.-%.

Die Zusammensetzung enthält in einer weiteren bevorzugten Ausführungsform eine Kombination aus mindestens einem Amidin der Formel (I) und mindestens einer Organozinn-Verbindung, insbesondere einer Diorganozinn(IV)-Verbindung wie die vorgängig genannten. Eine solche Zusammensetzung weist bereits bei einem geringen Zinn-Gehalt eine hohe Aushärtungsgeschwindigkeit auf, was aus toxikologischen und ökologischen Gründen vorteilhaft ist.

In einer Ausführungsform enthält die Zusammensetzung neben dem Amidin der Formel (I) zusätzlich mindestens ein Organotitanat. Eine Kombination aus einem Amidin der Formel (I) und einem Organotitanat weist eine besonders hohe katalytische Aktivität auf. Dadurch wird eine schnelle Aushärtung bei einer verhältnismässig geringen Einsatzmenge an Organotitanat ermöglicht. Als Organotitanat geeignet sind insbesondere Titan(IV)-Komplexverbindungen. Bevorzugte Organoititanate sind insbesondere ausgewählt aus
- Titan(IV)-Komplexverbindungen mit zwei 1,3-Diketonat-Liganden, insbesondere 2,4-Pentandionat (=Acetylacetonat), und zwei Alkoholat-Liganden;
- Titan(IV)-Komplexverbindungen mit zwei 1,3-Ketoesterat-Liganden, insbesondere Ethylacetoacetat, und zwei Alkoholat-Liganden;
- Titan(IV)-Komplexverbindungen mit einem oder mehreren Aminoalkoholat-Liganden, insbesondere Triethanolamin oder 2-((2-Aminoethyl)amino)ethanol, und einem oder mehreren Alkoholat-Liganden;
- Titan(IV)-Komplexverbindungen mit vier Alkoholat-Liganden;
- sowie höherkondensierte Organotitanate, insbesondere oligomeres Titan(IV)-tetrabutanolat, auch als Polybutyltitanat bezeichnet;
wobei als Alkoholat-Liganden insbesondere Isobutoxy, n-Butoxy, Isopropoxy, Ethoxy und 2-Ethylhexoxy geeignet sind.

Insbesondere geeignet sind die kommerziell erhältlichen Typen Tyzor^{®} AA, GBA, GBO, AA-75, AA-65, AA-105, DC, BEAT, BTP, TE, TnBT, KTM, TOT, TPT oder IBAY (alle von Dorf Ketal); Tytan PBT, TET, X85, TAA, ET, S2, S4 oder S6 (alle von Borica Company Ltd.) und Ken-React^{®} KR^{®} TTS, 7, 9QS, 12, 26S, 33DS, 38S, 39DS, 44, 134S, 138S, 133DS, 158FS oder LICA^{®} 44 (alle von Kenrich Petrochemicals).

Ganz besonders geeignete Organotitanate sind ausgewählt aus Bis(ethylacetoacetato)diisobutoxy-titan(IV) (kommerziell erhältlich beispielsweise als Tyzor^{®} IBAY von Dorf Ketal), Bis(ethylacetoacetato)diisopropoxy-titan(IV) (kommerziell erhältlich beispielsweise als Tyzor^{®} DC von Dorf Ketal), Bis(acetylacetonato)diisopropoxy-titan(IV), Bis(acetylacetonato)diisobutoxy-titan(IV), Tris(oxyethyl)-amin-isopropoxy-titan(IV), Bis[tris(oxyethyl)amin]diisopropoxy-titan(IV), Bis(2-ethylhexan-1,3-dioxy)-titan(IV), Tris[2-((2-Aminoethyl)amino)ethoxy]ethoxy-titan(IV), Bis(neopentyl(diallyl)oxy)-diethoxy-titan(IV), Titan(IV)-tetrabutanolat, Tetra(2-ethylhexyloxy)titanat, Tetra(isopropoxy)titanat und Polybutyltitanat. Am meisten bevorzugt sind Bis(ethylacetoacetato)diisobutoxy-titan(IV) oder Bis(ethylacetoacetato)diisopropoxy-titan(IV).

Die Zusammensetzung kann weitere Bestandteile enthalten, insbesondere die folgenden Hilfs- und Zusatzmittel:
- Haftvermittler und/oder Vernetzer, insbesondere Aminosilane wie insbesondere 3-Aminopropyl-trimethoxysilan, 3-Aminopropyl-dimethoxymethylsilan, N-(2-Aminoethyl)-3-aminopropyl-trimethoxysilan, N-(2-Aminoethyl)-3-aminopropyl-dimethoxymethylsilan, N-(2-Aminoethyl)-N'-[3-(trimethoxysilyl)propyl]ethylendiamin oder deren Analoga mit Ethoxy- anstelle von Methoxygruppen, weiterhin N-Phenyl-, N-Cyclohexyl- oder N-Alkylaminosilane, Mercaptosilane, Epoxysilane, (Meth)acrylosilane, Anhydridosilane, Carbamatosilane, Alkylsilane oder Iminosilane, oligomere Formen dieser Silane, Addukte aus primären Aminosilanen mit Epoxysilanen oder (Meth)acrylosilanen oder Anhydridosilanen, aminofunktionelle Alkylsilsesquioxane, insbesondere aminofunktionelles Methylsilsesquioxan oder aminofunktionelles Propylsilsesquioxan. Insbesondere geeignet sind 3-Aminopropyltrimethoxysilan, 3-Aminopropyltriethoxysilan, N-(2-Aminoethyl)-3-aminopropyltrimethoxysilan, N-(2-Aminoethyl)-3-aminopropyl-triethoxysilan, 3-Glycidoxypropyltrimethoxysilan, 3-Glycidoxypropyltriethoxysilan oder 3-Ureidopropyltrimethoxysilan, oder oligomere Formen dieser Silane;
- Trocknungsmittel, insbesondere Tetraethoxysilan, Vinyltrimethoxysilan, Vinyltriethoxysilan oder Organoalkoxysilane, welche in α-Stellung zur Silangruppe eine funktionelle Gruppe aufweisen, insbesondere N-(Methyldimethoxysilylmethyl)-O-methyl-carbamat, (Methacryloxymethyl)silane, Methoxymethylsilane, Orthoameisensäureester, Calciumoxid oder Molekularsiebe, insbesondere Vinyltrimethoxysilan oder Vinyltriethoxysilan;
- Weichmacher, insbesondere Trialkylsilyl-terminierte Polydialkylsiloxane, vorzugsweise Trimethylsilyl-terminierte Polydimethylsiloxane, insbesondere mit Viskositäten im Bereich von 10 bis 1'000 mPa s, oder entsprechende Verbindungen, bei denen einige der Methylgruppen ersetzt sind durch andere organische Gruppen, insbesondere Phenyl-, Vinyl- oder Trifluorpropylgruppen, sogenannte Reaktivweichmacher in Form von monofunktionellen, also einseitig reaktiven, Polysiloxanen, Carbonsäureester wie Phthalate, insbesondere Dioctylphthalat, Bis(2-ethylhexyl)phthalat, Bis(3-propylheptyl)-phthalat, Diisononylphthalat oder Diisodecylphthalat, Diester von ortho-Cyclohexandicarbonsäure, insbesondere Diisononyl-1,2-cyclohexandicarboxylat, Adipate, insbesondere Dioctyladipat, Bis(2-Ethylhexyl)adipat, Azelate, insbesondere Bis(2-ethylhexyl)acelat, Sebacate, insbesondere Bis(2-ethylhexyl)sebacat oder Diisononylsebacat, Polyole, insbesondere Polyoxyalkylenpolyole oder Polyesterpolyole, Glykolether, Glykolester, organische Phosphor- oder Sulfonsäureester, Sulfonsäureamide, Polybutene oder von natürlichen Fetten oder Ölen abgeleitete Fettsäuremethyl- oder -ethylester, auch "Biodiesel" genannt, wobei Siloxangruppen-haltige Weichmacher besonders geeignet sind für Silangruppen-haltige Polymere in Form von Polyorganosiloxanen;
- Lösemittel;
- anorganische oder organische Füllstoffe, insbesondere natürliche, gemahlene oder gefällte Calciumcarbonate, welche gegebenenfalls mit Fettsäuren, insbesondere Stearinsäure, beschichtet sind, Baryt (Schwerspat), Talke, Quarzmehle, Quarzsand, Dolomite, Wollastonite, Kaoline, calcinierte Kaoline, Mica (Kalium-Aluminium-Silikat), Molekularsiebe, Aluminiumoxide, Aluminiumhydroxide, Magnesiumhydroxid, Kieselsäuren inklusive hochdisperse Kieselsäuren aus Pyrolyseprozessen, industriell hergestellte Russe, Graphit, Metall-Pulver wie Aluminium, Kupfer, Eisen, Silber oder Stahl, PVC-Pulver oder Hohlkugeln;
- Fasern, insbesondere Glasfasern, Kohlefasern, Metallfasern, Keramikfasern oder Kunststofffasern wie Polyamidfasern oder Polyethylenfasern;
- Farbstoffe;
- Pigmente, insbesondere Titandioxid oder Eisenoxide;
- Rheologie-Modifizierer, insbesondere Verdickungsmittel, insbesondere Schichtsilikate wie Bentonite, Derivate von Rizinusöl, hydriertes Rizinusöl, Polyamide, Polyurethane, Harnstoffverbindungen, pyrogene Kieselsäuren, Celluloseether oder hydrophob modifizierte Polyoxyethylene;
- Stabilisatoren gegen Oxidation, Wärme, Licht oder UV-Strahlung;
- natürliche Harze, Fette oder Öle wie Kolophonium, Schellack, Leinöl, Rizinusöl oder Sojaöl;
- nicht-reaktive Polymere, wie insbesondere Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere Polyethylene (PE), Polypropylene (PP), Polyisobutylene, Ethylenvinylacetat-Copolymere (EVA) oder ataktische Poly-α-Olefine (APAO);
- flammhemmende Substanzen, insbesondere die bereits genannten Füllstoffe Aluminiumhydroxid und Magnesiumhydroxid, oder insbesondere organische Phosphorsäureester wie insbesondere Triethylphosphat, Trikresylphosphat, Triphenylphosphat, Diphenylkresylphosphat, Isodecyldiphenylphosphat, Tris(1,3-dichlor-2-propyl)phosphat, Tris(2-chlorethyl)phosphat, Tris(2-ethylhexyl)phosphat, Tris(chlorisopropyl)phosphat, Tris(chlorpropyl)-phosphat, isopropyliertes Triphenylphosphat, Mono-, Bis- oder Tris(isopropylphenyl)phosphate unterschiedlichen Isopropylierungsgrades, Resorcinol-bis(diphenylphosphat), Bisphenol-A-bis(diphenylphosphat) oder Ammoniumpolyphosphate;
- oberflächenaktive Substanzen, insbesondere Netzmittel, Verlaufsmittel, Entlüftungsmittel oder Entschäumer;
- Biozide, insbesondere Algizide, Fungizide oder das Pilzwachstum hemmende Substanzen;
sowie weitere üblicherweise in härtbaren Zusammensetzungen eingesetzte Substanzen. Es kann sinnvoll sein, gewisse Bestandteile vor dem Einmischen in die Zusammensetzung chemisch oder physikalisch zu trocknen.

In einer bevorzugten Ausführungsform enthält die Zusammensetzung mindestens ein Trocknungsmittel und mindestens einen Haftvermittler und/oder Vernetzer.

In einer bevorzugten Ausführungsform enthält die Zusammensetzung keine Phthalate als Weichmacher. Solche Zusammensetzungen sind toxikologisch vorteilhaft und weisen weniger Probleme mit Migrationseffekten auf.

Die Zusammensetzung wird vorzugsweise unter Ausschluss von Feuchtigkeit hergestellt und aufbewahrt. Typischerweise ist sie in einer geeigneten Verpackung oder Anordnung, wie insbesondere einer Flasche, einer Büchse, einem Beutel, einem Eimer, einem Fass oder einer Kartusche, unter Ausschluss von Feuchtigkeit lagerstabil.

Die Zusammensetzung kann in Form einer einkomponentigen oder in Form einer mehrkomponentigen, insbesondere zweikomponentigen, Zusammensetzung vorliegen.

Als "einkomponentig" wird im vorliegenden Dokument eine Zusammensetzung bezeichnet, bei welcher alle Bestandteile der Zusammensetzung vermischt im gleichen Gebinde gelagert werden und welche mit Feuchtigkeit härtbar ist. Als "zweikomponentig" wird im vorliegenden Dokument eine Zusammensetzung bezeichnet, bei welcher die Bestandteile der Zusammensetzung in zwei verschiedenen Komponenten vorliegen, welche in voneinander getrennten Gebinden gelagert werden. Erst kurz vor oder während der Applikation der Zusammensetzung werden die beiden Komponenten miteinander vermischt, worauf die vermischte Zusammensetzung aushärtet, gegebenenfalls unter Einwirkung von Feuchtigkeit.

Für den Fall, dass die Zusammensetzung ein Polyorganosiloxan mit endständigen Silangruppen enthält, ist sowohl eine einkomponentige Zusammensetzung, auch als RTV-1 bezeichnet, als auch eine zweikomponentige Zusammensetzung, auch als RTV-2 bezeichnet, bevorzugt. Im Fall einer RTV-2 Zusammensetzung ist das Polyorganosiloxan mit endständigen Silangruppen bevorzugt ein Bestandteil der ersten Komponente und ein Silanvernetzer, insbesondere ein Silanvernetzer der Formel (VI), ist bevorzugt ein Bestandteil der zweiten Komponente. Das Amidin der Formel (I) kann dabei in der ersten und/oder in der zweiten Komponente enthalten sein.

Für den Fall, dass die Zusammensetzung ein Silangruppen-haltiges organisches Polymer enthält, ist die Zusammensetzung bevorzugt einkomponentig.

Eine zweite oder gegebenenfalls weitere Komponenten wird bzw. werden mit der ersten Komponente vor oder bei der Applikation vermischt, insbesondere über einen Statikmischer oder über einen dynamischen Mischer.

Die Zusammensetzung wird insbesondere umgebungswarm, bevorzugt in einem Temperaturbereich zwischen 0°C und 45°C, insbesondere 5°C bis 35°C, appliziert und härtet auch bei diesen Bedingungen aus.

Bei der Applikation beginnt die Vernetzungsreaktion der Silangruppen, gegebenenfalls unter Einfluss von Feuchtigkeit. Vorhandene Silangruppen können mit vorhandenen Silanolgruppen zu Siloxangruppen (Si-O-Si-Gruppen) kondensieren. Vorhandene Silangruppen können bei Kontakt mit Feuchtigkeit auch zu Silanolgruppen (Si-OH-Gruppen) hydrolysieren und durch nachfolgende Kondensationsreaktionen Siloxangruppen (Si-O-Si-Gruppen) bilden. Als Ergebnis dieser Reaktionen härtet die Zusammensetzung schliesslich aus. Das Amidin der Formel (I) beschleunigt diese Aushärtung.

Falls für die Aushärtung Wasser benötigt wird, kann dieses entweder aus der Luft stammen (Luftfeuchtigkeit), oder aber die Zusammensetzung kann mit

einer Wasser enthaltenden Komponente in Kontakt gebracht werden, zum Beispiel durch Bestreichen, beispielsweise mit einem Abglättmittel, oder durch Besprühen, oder es kann der Zusammensetzung bei der Applikation Wasser oder eine Wasser enthaltende Komponente zugesetzt werden, zum Beispiel in Form einer wasserhaltigen oder Wasser freisetzenden Flüssigkeit oder Paste. Eine

Paste ist insbesondere geeignet für den Fall, dass die Zusammensetzung selber in Form einer Paste vorliegt.

Bei einer Aushärtung mittels Luftfeuchtigkeit härtet die Zusammensetzung von aussen nach innen durch, wobei zunächst eine Haut an der Oberfläche der Zusammensetzung gebildet wird. Die sogenannte Hautbildungszeit stellt ein Mass für die Aushärtungsgeschwindigkeit der Zusammensetzung dar. Die Geschwindigkeit der Aushärtung wird dabei im Allgemeinen von verschiedenen Faktoren, wie beispielsweise der Verfügbarkeit von Wasser, der Temperatur usw., bestimmt.

Die Zusammensetzung eignet sich für eine Vielzahl von Anwendungen, insbesondere als Anstrich, Lack oder Primer, als Harz zur Herstellung von Faserverbundwerkstoff (Composite), als Hartschaum, Weichschaum, Formteil, Elastomer, Faser, Folie oder Membran, als Vergussmasse, Dichtstoff, Klebstoff, Belag, Beschichtung oder Anstrich für Bau- und Industrieanwendungen, beispielsweise als Nahtabdichtung, Hohlraumversiegelung, Elektroisolationsmasse, Spachtelmasse, Fugendichtstoff, Schweiss- oder Bördelnahtdichtstoff, Montageklebstoff, Karrosserieklebstoff, Scheibenklebstoff, Sandwichelementklebstoff, Kaschierklebstoff, Laminatklebstoff, Verpackungsklebstoff, Holzklebstoff, Parkettklebstoff, Verankerungsklebstoff, Bodenbelag, Bodenbeschichtung, Balkonbeschichtung, Dachbeschichtung, Betonschutzbeschichtung, Parkhausbeschichtung, Versiegelung, Rohrbeschichtung, Korrosionsschutzbeschichtung, Textilbeschichtung, Dämpfungselement, Dichtungselement oder Spachtelmasse.

Besonders geeignet ist die Zusammensetzung als Klebstoff und/oder Dichtstoff, insbesondere für die Fugenabdichtung und für elastische Klebeverbindungen in Bau- und Industrieanwendungen, sowie als elastische Beschichtung mit rissüberbrückenden Eigenschaften, insbesondere zum Schützen und/oder Abdichten von beispielsweise Dächern, Böden, Balkonen, Parkdecks oder Betonröhren.

Bevorzugt stellt die Zusammensetzung somit einen Klebstoff oder einen Dichtstoff oder eine Beschichtung dar.

Eine solche Zusammensetzung enthält typischerweise Weichmacher, Füllstoffe, Haftvermittler und/oder Vernetzer und Trocknungsmittel und gegebenenfalls weitere Hilfs- und Zusatzstoffe.

Für eine Anwendung als Klebstoff oder Dichtstoff weist die Zusammensetzung bevorzugt eine pastöse Konsistenz mit strukturviskosen Eigenschaften auf. Ein solcher pastöser Dichtstoff oder Klebstoff wird insbesondere aus handelsüblichen Kartuschen, welche manuell, mittels Druckluft oder Batterie betrieben werden, oder aus einem Fass oder Hobbock mittels einer Förderpumpe oder eines Extruders, gegebenenfalls mittels eines Applikationsroboters, auf ein Substrat aufgetragen.

Für eine Anwendung als Beschichtung weist die Zusammensetzung bevorzugt eine bei Raumtemperatur flüssige Konsistenz mit selbstverlaufenden Eigenschaften auf. Gegebenenfalls ist sie leicht thixotrop, so dass die Beschichtung an abschüssigen bis senkrechten Flächen applizierbar ist, ohne sofort wegzufliessen. Sie wird insbesondere appliziert mittels Rolle oder Pinsel oder durch Ausgiessen und Verteilen mittels beispielsweise einem Roller, einem Schaber oder einer Zahntraufel.

Bei der Applikation wird die Zusammensetzung bevorzugt auf mindestens ein Substrat appliziert.

Geeignete Substrate sind insbesondere
- Glas, Glaskeramik, Beton, Mörtel, Backstein, Ziegel, Gips oder Natursteine wie Kalkstein, Granit oder Marmor;
- Metalle und Legierungen, wie Aluminium, Eisen, Stahl oder Buntmetalle, sowie oberflächenveredelte Metalle oder Legierungen, wie verzinkte oder verchromte Metalle;
- Leder, Textilien, Papier, Holz, mit Harzen, beispielsweise Phenol-, Melamin-oder Epoxidharzen, gebundene Holzwerkstoffe, Harz-Textil-Verbundwerkstoffe und weitere sogenannte Polymer-Composites;
- Kunststoffe, wie Polyvinylchlorid (Hart- und Weich-PVC), Acrylonitril-Butadien-Styrol-Copolymere (ABS), Polycarbonat (PC), Polyamid (PA), Polyester, Poly(methylmethacrylat) (PMMA), Epoxidharze, Polyurethane (PUR), Polyoxymethylen (POM), Polyolefine (PO), Polyethylen (PE) oder Polypropylen (PP), Ethylen/Propylen-Copolymere (EPM) oder Ethylen/Propylen/Dien-Terpolymere (EPDM), oder faserverstärkte Kunststoffe wie Kohlefaserverstärkte Kunststoffe (CFK), Glasfaser-verstärkte Kunststoffe (GFK) oder Sheet Moulding Compounds (SMC), wobei die Kunststoffe bevorzugt mittels Plasma, Corona oder Flammen oberflächenbehandelt sein können;
- beschichtete Substrate, wie pulverbeschichtete Metalle oder Legierungen;
- Farben oder Lacke, insbesondere Automobildecklacke.

Die Substrate können bei Bedarf vor dem Applizieren der Zusammensetzung vorbehandelt werden, insbesondere durch physikalische und/oder chemische Reinigungsverfahren oder durch das Aufbringen eines Haftvermittlers, einer Haftvermittlerlösung oder eines Primers.

Besonders geeignet ist die Zusammensetzung für den Kontakt mit Substraten, die besonders empfindlich sind auf Störungen durch migrierende Substanzen, insbesondere durch das Ausbilden von Verfärbungen oder Flecken. Dies sind insbesondere feinporige Substrate wie Marmor, Kalkstein oder andere Natursteine, Gips, Zementmörtel oder Beton, aber auch Kunststoffe. Insbesondere auf PVC werden bei der Anwesenheit von Katalysatoren wie beispielsweise DBU oder TMG starke Verfärbungen beobachtet, die sich durch Reinigung nicht entfernen lassen. Solche Effekte werden mit dem Amidin der Formel (I) nicht beobachtet.

Verklebt oder abgedichtet werden können zwei gleichartige oder zwei verschiedene Substrate, insbesondere die vorgängig genannten Substrate.

Nach der Aushärtung der Zusammensetzung mit Wasser, insbesondere in Form von Luftfeuchtigkeit, und/oder mit mindestens einem geeigneten Vernetzer wird eine ausgehärtete Zusammensetzung erhalten.

Aus der Anwendung der Zusammensetzung entsteht ein Artikel, welcher mit der Zusammensetzung insbesondere verklebt, abgedichtet oder beschichtet wurde. Beim Artikel handelt es sich insbesondere um ein Bauwerk, insbesondere ein Bauwerk des Hoch- oder Tiefbaus, um ein industriell gefertigtes Gut oder um ein Konsumgut, insbesondere ein Fenster, eine Haushaltmaschine oder ein Transportmittel wie insbesondere ein Automobil, ein Bus, ein Lastkraftwagen, ein Schienenfahrzeug, ein Schiff, ein Flugzeug oder ein Helikopter; oder der Artikel kann ein Anbauteil davon sein.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

Als "Normklima" wird eine Temperatur von 23±1°C und eine relative Luftfeuchtigkeit von 50±5% bezeichnet.

"EEW" steht für das Epoxid-Equivalentgewicht.

**¹H-NMR-Spektren** wurden auf einem Spektrometer des Typs Bruker Ascend 400 bei 400.14 MHz gemessen; die chemischen Verschiebungen δ sind angegeben in ppm relativ zu Tetramethylsilan (TMS). Kopplungskonstanten J sind angegeben in Hz. Echte und Pseudo-Kopplungsmuster wurden nicht unterschieden.

**Infrarotspektren** (FT-IR) wurden auf einem mit horizontaler ATR-Messeinheit mit Diamant-Kristall ausgestatteten FT-IR Gerät Nicolet iS5 von Thermo Scientific gemessen. Flüssige Proben wurden unverdünnt als Filme aufgetragen, feste Proben wurden in CH₂Cl₂ gelöst. Die Absorptionsbanden sind in Wellenzahlen (cm⁻¹) angegeben (Messfenster: 4000-650 cm⁻¹).

Die **Hautbildungszeit** (HBZ) wurde dadurch bestimmt, dass einige Gramm der Zusammensetzung in einer Schichtdicke von ca. 2 mm auf Pappkarton aufgetragen wurden und im Normklima die Zeitdauer gemessen wurde, bis beim leichten Antippen der Oberfläche der Zusammensetzung mittels einer Pipette aus LDPE erstmals keine Rückstände auf der Pipette mehr zurückblieben.

Die Beschaffenheit der **Oberfläche** wurde haptisch geprüft.

Die mechanischen Eigenschaften **Zugfestigkeit, Bruchdehnung** und **E-Modul** (bei 0-5% sowie bei 0-50% bzw. 0-100% Dehnung) wurden gemäss DIN EN 53504 bei einer Zuggeschwindigkeit von 200 mm/min. gemessen.

### verwendete funktionelle Verbindungen:

| | |
|---|---|
| PGE | Phenylglycidylether (von Sigma-Aldrich) |
| GLYEO | 3-Glycidoxypropyltriethoxysilan (Dynasylan^{®} GLYEO, von Evonik) |
| TBA | tert.Butylacrylat (von Sigma-Aldrich) |
| THFMA | Tetrahydrofurfurylmethacrylat (von Sigma-Aldrich) |
| TMPTA | 1,1,1-Trimethylolpropantriacrylat (SR-351, von Sartomer) |
| Y-9936 | 3-Methacryloxypropyltriethoxysilan (Silquest^{®} Y-9936, von Momentive) |
| NAM | 4-Acryloylmorpholin (von Sigma-Aldrich) |
| DEM | Diethylmaleat (von Sigma-Aldrich) |

### Herstellung von Amidinen der Formel HZ:

### Amidin HZ-1: 2-Methyl-1,4,5,6-tetrahydropyrimidin

In einem Rundkolben wurden 7.58 g 1,3-Propandiamin, 16.37 g Trimethylorthoacetat und 0.60 g Lanthan(III)-trifluormethansulfonat vermischt und unter Rühren während 24 Stunden auf 100 °C erwärmt. Darauf wurde das Reaktionsgemisch im Vakuum von den flüchtigen Bestandteilen befreit. Man erhielt 5.97 g eines weissen Feststoffs.

¹H-NMR (CDCl₃): δ 1.75 (*quint,* 2 H, J = 5.8, NCH₂C*H*₂CH₂N), 1.83 (s, 3 H, CH₃), 3.30 (t, 4 H, J = 5.8, NCH₂).

FT-IR: 3214, 3177, 2996, 2925, 2843, 1630, 1542, 1475, 1438, 1380, 1360, 1322, 1294, 1273, 1204, 1191, 1139, 1114, 1095, 1035, 1009, 977, 915, 875, 839, 731.

### Herstellung von Amidinen der Formel (I):

### Amidin K-1 (nicht erfindungsgemäss): 1-(2-Hydroxy-3-phenoxyprop-1-yl)-2-methyl-1,4,5,6-tetrahydropyrimidin

In einem Rundkolben wurden 1.26 g Amidin HZ-1 mit 1.93 g PGE vermischt und während 3 Stunden auf 80 °C erwärmt. Man erhielt ein geruchloses, gelbes, hochviskoses Öl.

FT-IR: 3060, 2925, 2852, 2714, 1598, 1586, 1494, 1430, 1379, 1359, 1316, 1299, 1241, 1172, 1153, 1077, 1036, 1020, 951, 918, 881, 814, 751, 690.

### Amidin K-2: 1-(2-Hydroxy-3-(3-triethoxysilylpropoxy)prop-1-yl)-2-methyl-1,4,5,6-tetrahydropyrimidin

In einem Rundkolben wurden 1.55 g Amidin HZ-1 mit 4.21 g GLYEO vermischt und auf 100 °C erwärmt, bis nach 3.5 Stunden mittels Gaschromatographie kein GLYEO mehr nachweisbar war. Man erhielt ein geruchloses gelbes Öl.

¹H-NMR (CDCl₃): δ 0.62 (br *m*, 2 H, CH₂Si), 1.20 (*t*, 9 H, J = 6.8, CH₂C*H₃*), 1.6-1.75 (*m*, 2 H, C*H₂*CH₂Si), 1.77-1.85 (*m*, 2 H, NCH₂C*H*₂CH₂N), 1.99 (*s*, 3 H, N=CCH₃), 3.22-3.3 (*m*, 6 H, C*H₂*N), 3.3-3.5 und 3.65 (*m,* 4 H, C*H₂*OC*H₂*), 3.81 (*q*, 6 H, J = 6.9, SiOCH₂), 4.16 (br *m*, 1 H, CHOH).

FT-IR: 2971, 2925, 2882, 2863, 1615, 1550, 1482, 1433, 1379, 1318, 1294, 1261, 1194, 1164, 1100, 1074, 1031, 951, 880, 850, 772, 655.

### Amidin K-3 (nicht erfindungsgemäss): tert.Butyl 3-(2-methyl-1,4,5,6-tetrahydropyrimidin-1-yl)propionat

In einem Rundkolben wurden 2.20 g Amidin HZ-1 mit 2.72 g TBA vermischt und auf 100 °C erwärmt, bis nach 5 Stunden mittels Gaschromatographie kein TBA mehr nachweisbar war. Darauf wurde das Reaktionsgemisch im Vakuum von den flüchtigen Bestandteilen befreit. Man erhielt ein geruchloses gelbes Öl. ¹H-NMR (CDCl₃): δ 1.47 (*s*, 9 H, C(CH₃)₃), 1.81 (*t*, 2 H, J = 5.8, NCH₂C*H*₂CH₂N), 1.99 (s, 3 H, N=CCH₃), 2.44 (*t*, 2 H, J = 7.3, CH₂C=O), 3.17 (*t,* 2 H, J = 5.9, NCH₂^{Cy}), 3.27-3.32 (*m*, 2 H, NCH₂^{Cy}), 3.45 (*t*, 2 H, J = 7.2, N*CH₂*CH₂C=O).

FT-IR: 3220, 2974, 2929, 2848, 1723, 1669, 1618, 1551, 1422, 1366, 1316, 1281, 1253, 1211, 1150, 1119, 1083, 1046, 1008, 988, 953, 922, 882, 845, 753, 695.

### Amidin K-4 (nicht erfindungsgemäss): Tetrahydrofurfuryl 3-(2-methyl-1,4,5,6-tetrahydropyrimidin-1-yl)-2-methylpropionat

In einem Rundkolben wurden 2.03 g Amidin HZ-1 mit 3.36 g THFMA vermischt und auf 100°C erwärmt, bis nach 5 Stunden mittels Gaschromatographie kein THFMA mehr nachweisbar war. Darauf wurde das Reaktionsgemisch im Vakuum von den flüchtigen Bestandteilen befreit. Man erhielt ein geruchloses oranges Öl.

FT-IR: 3292, 2930, 2860, 1732, 1696, 1638, 1615, 1564, 1496, 1440, 1377, 1361, 1319, 1287, 1259, 1208, 1173, 1140, 1068, 1031, 986, 952, 919, 885, 841, 814, 724, 688.

### Amidin K-5 (nicht erfindungsgemäss): Reaktionsgemisch enthaltend 1,1,1-Trimethylolpropan bis(3-(2-methyl-1,4,5,6-tetrahydropyrimidin-1-yl)propionat) acrylat

In einem Rundkolben wurden 2.45 g Amidin HZ-1, 3.56 g TMPTA und 10 ml Tetrahydrofuran vermischt und auf 80 °C erwärmt, bis nach 24 Stunden mittels Gaschromatographie kein TMPTA mehr nachweisbar war. Darauf wurde das Reaktionsgemisch im Vakuum von den flüchtigen Bestandteilen befreit. Man erhielt ein geruchloses, gelbes, hochviskoses Öl.

FT-IR: 3258, 2928, 2853, 1729, 2696, 1637, 1613, 1554, 1494, 1443, 1382, 1319, 1284, 1206, 1171, 1119, 1048, 1030, 992, 949, 914, 885, 852, 841, 778, 713, 686.

### Amidin K-6 (nicht erfindungsgemäss): 3-Triethoxysilylpropyl 3-(2-methyl-1,4,5,6-tetrahydropyrimidin-1-yl)-2-methylpropionat

In einem Rundkolben wurden 1.97 g Amidin HZ-1, 5.15 g Y-9936 und 8 ml Tetrahydrofuran vermischt und während 6 Stunden auf 90°C erwärmt. Darauf wurde das Reaktionsgemisch im Vakuum von den flüchtigen Bestandteilen befreit. Man erhielt ein geruchloses gelbes Öl.

FT-IR: 3180, 2971, 2927, 2883, 1731, 1720, 1637, 1619, 1566, 1497, 1440, 1383, 1319, 1295, 1254, 1166, 1075, 1018, 951, 886, 841, 765, 690.

### Amidin K-7 (nicht erfindungsgemäss): 3-(2-Methyl-1,4,5,6-tetrahydropyrimidin-1-yl)-1-morpholinopropan-1-on

In einem Rundkolben wurden 1.16 g Amidin HZ-1 und 1.68 g NAM vermischt und während 4 Stunden auf 80 °C erwärmt. Man erhielt ein geruchloses gelbes Öl, welches beim Stehenlassen bei Raumtemperatur fest wurde.

¹H-NMR (CDCl₃): δ 1.80-1.87 (*m*, 2 H, NCH₂C*H*₂CH₂N), 2.01 (s, 3 H, CH₃), 2.53 (*t*, 2 H, J = 7.3, CH₂C=O), 3.16-3.32 und 3.27-3.34 (2 x *m*, 2 x 2 H, NC*H*₂CH₂C*H*₂N), 3.44-3.5 (*m*, 2 H, CH₂N^{Morpholin}), 3.53 (*t*, 2 H, J = 7.5, N*CH₂*CH₂C=O), 3.6-3.65 (*m*, 2 H, CH₂N^{Morpholin}), 3.66-3.7 (*m*, 4 H, CH₂O). FT-IR: 3177, 2924, 2851, 1611, 1424, 1380, 1358, 1316, 1298, 1270, 1230, 1214, 1113, 1085, 1068, 1025, 1008, 982, 845, 915, 882, 847, 790, 720.

### Amidin K-8 (nicht erfindungsgemäss): Diethyl 2-(2-methyl-1,4,5,6-tetrahydropyrimidin-1-yl)succinat

In einem Rundkolben wurden 1.84 g Amidin HZ-1 und 3.28 g DEM vermischt und auf 60 °C erwärmt, bis nach 1 Stunde mittels Dünnschichtchromatographie kein DEM mehr nachweisbar war. Man erhielt ein geruchloses oranges Öl. FT-IR: 3221, 2979, 2933, 2853, 1729, 1674, 1625, 1568, 1502, 1475, 1442, 1414, 1393, 1361, 1295, 1269, 1156, 1096, 1026, 974, 957, 882, 824, 792, 777, 735, 690.

### Herstellung von Silangruppen-haltigen Polyethern:

### Polymer STP-1:

Unter Feuchtigkeitsausschluss wurden 1000 g Polyol Acclaim^{®} 12200 (Polyoxypropylendiol mit niedrigem Ungesättigtheitsgrad, von Bayer; OH-Zahl 11.0 mg KOH/g), 43.6 g Isophorondiisocyanat (IPDI; Vestanat^{®} IPDI, von Evonik), 126.4 g Diisodecylphthalat (DIDP) und 0.1 g Bismuttris(neodecanoat) (10 Gew.-% in DIDP) unter stetigem Rühren auf 90 °C aufgeheizt und auf dieser Temperatur belassen, bis der titrimetrisch bestimmte Gehalt an freien Isocyanatgruppen einen stabilen Wert von 0.63 Gew.-% erreicht hatte. Anschliessend wurden 63.0 g N-(3-Trimethoxysilylpropyl)-amino-bernsteinsäurediethylester (Addukt aus 3-Aminopropyltrimethoxysilan und Maleinsäurediethylester; hergestellt nach den Angaben in US 5,364,955) eingemischt und die Mischung bei 90 °C solange gerührt, bis mittels FT-IR-Spektroskopie kein freies Isocyanat mehr nachgewiesen wurde. Der so erhaltene Trimethoxysilangruppen-haltige Polyether mit einem Silan-Equivalentgewicht von ca. 6880 g/Eq (aus den Einsatzmengen berechnet) wurde auf Raumtemperatur abgekühlt und unter Ausschluss von Feuchtigkeit aufbewahrt.

### Verwendete kommerzielle Katalysatoren:

| | |
|---|---|
| DBU | 1,8-Diazabicyclo[5.4.0]undec-7-en (Lupragen^{®} N 700, von BASF) |
| TMG | 1,1,3,3-Tetramethylguanidin (von Sigma-Aldrich) |

### Zusammensetzungen auf Basis von Silangruppen-haltigen Polymeren:

Vergleichsbeispiele sind in den Tabellen 1 bis 4 mit "(Ref)" gekennzeichnet.

### Zusammensetzungen Z1 bis Z10:

Eine Zusammensetzung aus 96.5 g Polymer STP-1, 0.5 g Vinyltrimethoxysilan und 3.0 g 3-Aminopropyltrimethoxysilan wurde mit verschiedenen Katalysatoren in der angegebenen Menge gemäss Tabelle 1 vermengt und die Mischung auf Viskosität und Hautbildungszeit (HBZ) im Normklima, vor und nach Lagerung, geprüft. Die Hautbildungszeit dient dabei als Mass für die Aktivität des Katalysators in Bezug auf die Vernetzungsreaktion der Silangruppen, d.h. für die Vernetzungsgeschwindigkeit; die Veränderung der Viskosität und der Hautbildungszeit nach Lagerung sind ein Mass für die Lagerstabilität. Weiterhin wurde die applizierte Mischung nach 24 Stunden im Normklima daraufhin geprüft, ob die Oberfläche wie gewünscht trocken war oder sich ein schmieriger Film gebildet hatte, was ein Anzeichen für das Ausschwitzen des Katalysators aufgrund schlechter Verträglichkeit mit dem ausgehärteten Kunststoff ist, und/oder ob die Oberfläche klebrig war, was ein Anzeichen für eine unvollständige Aushärtung ist. Weiterhin wurde von der Mischung ein Film von 2 mm Dicke hergestellt, während 7 Tagen im Normklima aushärten lassen und auf mechanische Eigenschaften geprüft. Die Ergebnisse sind in Tabelle 1 und 2 wiedergegeben. "Zus." steht für "Zusammensetzung".

**Tabelle 1:**

| **Zus.** | **Katalysator** | **Menge** | **Konzentration¹** | **Viskosität [Pa·s]** | | | **HBZ** | |
|---|---|---|---|---|---|---|---|---|
| | | | | **frisch** | **gelagert²** | **Zunahme** | **frisch** | **gelagert²** |
| **Z1** (Ref) | Amidin K-1³ | 0.46 g | 1.9 | 29.2 | 30.5 | 4% | 20' | 24' |
| **Z2** | Amidin K-2 | 0.69 g | 1.9 | 22.0 | 28.3 | 29% | 27' | 29' |
| **Z3** (Ref) | Amidin K-3 | 0.41 g | 1.9 | 21.3 | 25.5 | 20% | 29' | 32' |
| **Z4** (Ref) | Amidin K-4 | 0.49 g | 1.9 | 21.9 | 29.1 | 33% | 30' | 42' |
| **Z5** (Ref) | Amidin K-5 | 0.48 g | 1.9 | 30.6 | 35.4 | 16% | 53' | 54' |
| **Z6** (Ref) | Amidin K-6 | 0.71 g | 1.9 | 29.3 | 32.1 | 10% | 28' | 29' |
| **Z7** (Ref) | Amidin K-7³ | 0.44 g | 1.9 | 30.0 | 38.0 | 27% | 25' | 21' |
| **Z8** (Ref) | Amidin K-8 | 0.50 g | 1.9 | 30.3 | 34.5 | 14% | 29' | 30' |
| **Z9** (Ref) | DBU | 0.28 g | 1.9 | 27.2 | 36.9 | 36% | 25' | 29' |
| **Z10** (Ref) | TMG | 0.21 g | 1.9 | 22.3 | 24.6 | 10% | 65' | 75' |

| | | | | | | | | |
|---|---|---|---|---|---|---|---|---|
| ¹ mmol Amidin- oder Guanidin-Gruppen auf 100 g Silangruppen-haltiger Polyether. ² während 7 Tagen bei 60°C in geschlossenem Gebinde. ³ als Lösung (30 Gew.-%) in N-Ethylpyrrolidon. | | | | | | | | |

**Tabelle 2:**

| **Zus.** | **Oberfläche nach 24h** | **Zugfestigkeit** | **Bruchdehnung** | **E-Modul** | |
|---|---|---|---|---|---|
| | | | | **0-5%** | **0-50%** |
| **Z1** (Ref) | trocken | 0.67 MPa | 89% | 1.29 MPa | 0.82 MPa |
| **Z2** | fast trocken | 0.71 MPa | 100% | 1.23 MPa | 0.82 MPa |
| **Z3** (Ref) | fast trocken | 0.68 MPa | 95% | 1.25 MPa | 0.80 MPa |
| **Z4** (Ref) | fast trocken | 0.73 MPa | 110% | 1.25 MPa | 0.79 MPa |
| **Z5** (Ref) | trocken | 0.78 MPa | 126% | 1.21 MPa | 0.79 MPa |
| **Z6** (Ref) | trocken | 0.82 MPa | 99% | 1.26 MPa | 0.83 MPa |
| **Z7** (Ref) | trocken | 0.69 MPa | 88% | 1.22 MPa | 0.81 MPa |
| **Z8** (Ref) | trocken | 0.70 MPa | 95% | 1.17 MPa | 0.82 MPa |
| **Z9** (Ref) | schmierig | 0.58 MPa | 72% | 1.16 MPa | 0.77 MPa |
| **Z10** (Ref) | klebrig | 0.62 MPa | 90% | 1.19 MPa | 0.75 MPa |

### Zusammensetzungen Z11 und Z12

In einem Rundkolben wurden 71.1 g eines OH-terminierten linearen Polydimethylsiloxans mit einer Viskosität von ca. 50'000 mPas bei 23 °C (Wacker^{®} Silicone Rubber Polymer FD 50, von Wacker) mit 2.6 g Vinyl-tris(methylethylket-oximo)silan vermengt und während 15 Minuten unter Vakuum gerührt. In das so erhaltene Polydimethylsiloxan mit Vinyl-bis(methylethylketoximo)silyl-Endgruppen wurden 26.3 g Trimethylsilyl-terminiertes Polydimethylsiloxan (Wacker^{®} AK 100 Siliconöl, von Wacker) eingerührt. Diese Mischung wurde mit verschiedenen Katalysatoren gemäss nachstehender Tabelle 3 vermengt und wie für Zusammensetzung Z1 beschrieben auf Viskosität, Hautbildungszeit (HBZ), Oberflächenbeschaffenheit sowie mechanische Eigenschaften geprüft. Die Ergebnisse sind in Tabelle 3 und 4 wiedergegeben. "Zus." steht für "Zusammensetzung".

**Tabelle 3:**

| **Zus.** | **Katalysator** | **Menge** | **Konzentration¹** | **Viskosität [Pa·s]** | | | **HBZ** | |
|---|---|---|---|---|---|---|---|---|
| | | | | **frisch** | **gelagert²** | **Zunahme** | **frisch** | **gelagert²** |
| **Z11** | Amidin K-2 | 0.15 g | 0.6 | 13.1 | 9.6 | -27% | 23' | 27' |
| **Z12** (Ref) | DBU | 0.06 g | 0.6 | 13.0 | 10.2 | -22% | 13' | 14' |

| | | | | | | | | |
|---|---|---|---|---|---|---|---|---|
| ¹ mmol Amidin-Gruppen auf 100 g Ketoximato-Polydimethylsiloxan Polymer. ² während 7 Tagen bei 70°C in geschlossenem Gebinde. | | | | | | | | |

**Tabelle 4:**

| **Zus.** | **Oberfläche nach 24h** | **Zugfestigkeit** | **Bruchdehnung** | **E-Modul** | |
|---|---|---|---|---|---|
| | | | | **0-5%** | **0-50%** |
| **Z11** | trocken | 0.18 MPa | 240% | 0.20 MPa | 0.11 MPa |
| **Z12** (Ref) | fast trocken | 0.25 MPa | 289% | 0.22 MPa | 0.16 MPa |

## Patentansprüche

1. Amidin der Formel (I), 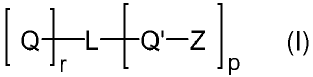 wobei
p für 1 und r für 0 steht,
L für einen mit einer Alkoxysilangruppe substituierten Alkylrest mit 1 bis 6 C-Atomen steht,
Q für Glycidoxy steht,
Q' für eine zweiwertige Verbindungseinheit entstanden aus der Reaktion einer Reaktivgruppe Q mit HZ steht, wobei
Q'für 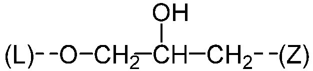 steht, und
Z für eine über ein Stickstoffatom gebundene aliphatische Amidingruppe steht.

2. Amidin gemäss Anspruch 1, **dadurch gekennzeichnet, dass** es der Formel (I') entspricht, 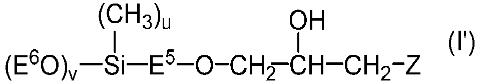 wobei
E⁵ für einen Alkylenrest mit 1 bis 6 C-Atomen steht,
E⁶ für einen Alkylrest mit 1 bis 4 C-Atomen, insbesondere für Methyl oder für Ethyl, steht, und
u für 0 oder 1 und v für 2 oder 3 stehen, wobei (u+v) für 3 steht, und
Z für eine über ein Stickstoffatom gebundene aliphatische Amidingruppe steht.

3. Amidin gemäss Anspruch 1 oder 2, **dadurch gekennzeichnet, dass** L für Triethoxysilylpropyl oder Trimethoxysilylpropyl steht.

4. Amidin gemäss einem der Ansprüche 1 bis 3, **dadurch gekennzeichnet, dass** Z für 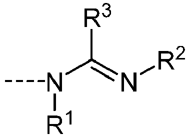 steht, wobei
R¹ für einen Wasserstoff-Rest oder für einen Alkyl- oder Cycloalkyl- oder Aralkyl-Rest mit 1 bis 8 C-Atomen oder zusammen mit R² für R⁴ steht, R² für einen Wasserstoff-Rest oder für einen Alkyl-, Cycloalkyl- oder Aralkyl-Rest mit 1 bis 18 C-Atomen, welcher gegebenenfalls Ether-Sauerstoff oder tertiären Amin-Stickstoff enthält, oder zusammen mit R¹ für R⁴ steht,
R³ für einen Wasserstoff-Rest oder für einen Alkyl- oder Cycloalkyl- oder Aralkyl-Rest mit 1 bis 12 C-Atomen steht,
wobei R⁴ für einen gegebenenfalls substituierten 1,2-Ethylen-, 1,3-Propylen- oder 1,4-Butylen-Rest mit 2 bis 12 C-Atomen steht,
und wobei
R² und R³ auch zusammen für einen Alkylen-Rest mit 3 bis 6 C-Atomen stehen können.

5. Amidin gemäss Anspruch 4, **dadurch gekennzeichnet, dass** R¹ und R² zusammen für R⁴ stehen.

6. Verfahren zur Herstellung des Amidins der Formel (I) gemäss einem der Ansprüche 1 bis 5, **dadurch gekennzeichnet, dass** mindestens ein Amidin der Formel HZ mit mindestens einer funktionellen Verbindung der Formel 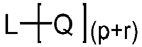 umgesetzt wird.

7. Verfahren gemäss Anspruch 6, **dadurch gekennzeichnet, dass** das Amidin der Formel HZ die Formel 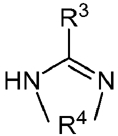 aufweist.

8. Verwendung des Amidins der Formel (I) gemäss einem der Ansprüche 1 bis 5 als Katalysator für die Vernetzung einer härtbaren Zusammensetzung.

9. Verwendung gemäss Anspruch 8, **dadurch gekennzeichnet, dass** die härtbare Zusammensetzung eine Epoxidharz-Zusammensetzung oder eine Polyurethan-Zusammensetzung oder eine Epoxidharz-Polyurethan-Zusammensetzung oder eine Cyanatesterharz-Zusammensetzung oder eine Silangruppen-haltige Zusammensetzung, insbesondere eine Polyurethan-Zusammensetzung oder eine Silangruppen-haltige Zusammensetzung, ist.

10. Verwendung gemäss Anspruch 9, **dadurch gekennzeichnet, dass** die härtbare Zusammensetzung eine Zusammensetzung auf Basis von Silangruppen-haltigen Polymeren ausgewählt aus der Gruppe bestehend aus Polyorganosiloxanen mit endständigen Silangruppen und Silangruppen-haltigen organischen Polymeren ist.

11. Härtbare Zusammensetzung enthaltend mindestens ein Amidin der Formel (I) gemäss einem der Ansprüche 1 bis 5.

12. Zusammensetzung gemäss Anspruch 11, **dadurch gekennzeichnet, dass** sie weiterhin mindestens ein Silangruppen-haltiges Polymer enthält.

13. Zusammensetzung gemäss einem der Ansprüche 11 bis 12, **dadurch gekennzeichnet, dass** sie einen Klebstoff oder einen Dichtstoff oder eine Beschichtung darstellt.

## Claims

1. Amidine of the formula (I) 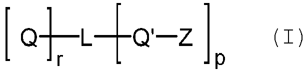 where
p is 1 and r is 0,
L is an alkyl radical substituted by an alkoxysilane group and having 1 to 6 carbon atoms,
Q is glycidoxy,
Q' is a divalent connecting unit formed from the reaction of a reactive Q group with HZ, where
Q' is 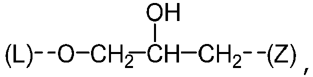 and
Z is an aliphatic amidine group bonded via a nitrogen atom.

2. Amidine according to Claim 1, **characterized in that** it conforms to the formula (I') 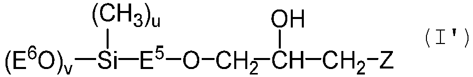 where
E⁵ is an alkylene radical having 1 to 6 carbon atoms,
E⁶ is an alkyl radical having 1 to 4 carbon atoms, especially methyl or ethyl, and
u is 0 or 1 and v is 2 or 3, where (u+v) is 3, and
Z is an aliphatic amidine group bonded via a nitrogen atom.

3. Amidine according to Claim 1 or 2, **characterized in that** L is triethoxysilylpropyl or trimethoxysilylpropyl.

4. Amidine according to any of Claims 1 to 3, **characterized in that** Z is 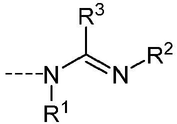 where
R¹ is a hydrogen radical or an alkyl or cycloalkyl or aralkyl radical having 1 to 8 carbon atoms or together with R² is R⁴,
R² is a hydrogen radical or an alkyl, cycloalkyl or aralkyl radical which has 1 to 18 carbon atoms and optionally contains ether oxygen or tertiary amine nitrogen, or together with R¹ is R⁴,
R³ is a hydrogen radical or an alkyl or cycloalkyl or aralkyl radical having 1 to 12 carbon atoms,
where R⁴ is an optionally substituted 1,2-ethylene, 1,3-propylene or 1,4-butylene radical having 2 to 12 carbon atoms,
and where
R² and R³ together may also be an alkylene radical having 3 to 6 carbon atoms.

5. Amidine according to Claim 4, **characterized in that** R¹ and R² together are R⁴.

6. Process for preparing the amidine of the formula (I) according to any of Claims 1 to 5, **characterized in that** at least one amidine of the formula HZ is reacted with at least one functional compound of the formula

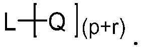

7. Process according to Claim 6, **characterized in that** the amidine of the formula HZ has the formula

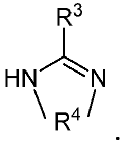

8. Use of the amidine of the formula (I) according to any of Claims 1 to 5 as catalyst for the crosslinking of a curable composition.

9. Use according to Claim 8, **characterized in that** the curable composition is an epoxy resin composition or a polyurethane composition or an epoxy resin/polyurethane composition or a cyanate ester resin composition or a composition containing silane groups, especially a polyurethane composition or a composition containing silane groups.

10. Use according to Claim 9, **characterized in that** the curable composition is a composition based on polymers containing silane groups selected from the group consisting of polyorganosiloxanes having terminal silane groups and organic polymers containing silane groups.

11. Curable composition comprising at least one amidine of the formula (I) according to any of Claims 1 to 5.

12. Composition according to Claim 11, **characterized in that** it further comprises at least one polymer containing silane groups.

13. Composition according to either of Claims 11 and 12, **characterized in that** it is an adhesive or a sealant or a coating.

## Revendications

1. Amidine de formule (I) 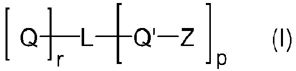 dans laquelle
p représente 1 et r représente 0,
L représente un radical alkyle comprenant 1 à 6 atomes de carbone, substitué par un groupe alcoxysilane,
Q représente glycidoxy,
Q' représente un motif de liaison bivalent formé par la réaction d'un groupe réactif Q avec HZ, où
Q' représente 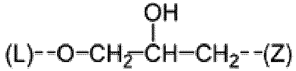 et
Z représente un groupe amidine aliphatique lié par l'intermédiaire d'un atome d'azote,

2. Amidine selon la revendication 1, **caractérisée en ce qu'**elle correspond à la formule (I'), 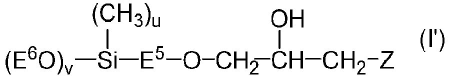 dans laquelle
E⁵ représente un radical alkylène comprenant 1 à 6 atomes de carbone,
E⁶ représente un radical alkyle comprenant 1 à 4 atomes de carbone, en particulier méthyle ou éthyle, et
u représente 0 ou 1 et v représente 2 ou 3, (u + v) représentant 3, et
Z représente un groupe amidine aliphatique lié par l'intermédiaire d'un atome d'azote.

3. Amidine selon la revendication 1 ou 2, **caractérisée en ce que** L représente triéthoxysilylpropyle ou triméthoxysilylpropyle.

4. Amidine selon l'une quelconque des revendications 1 à 3, **caractérisée en ce que**
Z représente 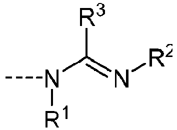
dans laquelle
R¹ représente un radical hydrogène ou un radical alkyle ou cycloalkyle ou aralkyle comprenant 1 à 8 atomes de carbone ou, conjointement avec R², représente R⁴,
R² représente un radical hydrogène ou un radical alkyle, cycloalkyle ou aralkyle comprenant 1 à 18 atomes de carbone qui contient le cas échéant un oxygène à fonction éther ou un azote à fonction amine tertiaire ou, conjointement avec R¹, représente R⁴,
R³ représente un radical hydrogène ou un radical alkyle ou cycloalkyle ou aralkyle comprenant 1 à 12 atomes de carbone,
R⁴ représentant un radical 1,2-éthylène, 1,3-propylène ou 1,4-butylène comprenant 2 à 12 atomes de carbone, le cas échéant substitué et
R² et R³ pouvant également représenter, ensemble, un radical alkylène comprenant 3 à 6 atomes de carbone.

5. Amidine selon la revendication 4, **caractérisée en ce que** ou R¹ et R² représentent ensemble R⁴.

6. Procédé pour la préparation de l'amidine de formule (I) selon l'une quelconque des revendications 1 à 5, **caractérisé en ce qu'**au moins une amidine de formule HZ est transformée avec au moins un composé fonctionnel de formule

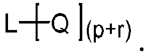

7. Procédé selon la revendication 6, **caractérisé en ce que** l'amidine de formule HZ présente la formule

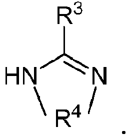

8. Utilisation de l'amidine de formule (I) selon l'une quelconque des revendications 1 à 5 comme catalyseur pour la réticulation d'une composition durcissable.

9. Utilisation selon la revendication 8, **caractérisée en ce que** la composition durcissable est une composition de résine époxyde ou une composition de polyuréthane ou une composition de résine époxyde-polyuréthane ou une composition de résine de cyanatoester ou une composition contenant des groupes silane, en particulier une composition de polyuréthane ou une composition contenant des groupes silane.

10. Utilisation selon la revendication 9, **caractérisée en ce que** la composition durcissable est une composition à base de polymères contenant des groupes silane choisis dans le groupe constitué par les polyorganosiloxanes présentant des groupes silane en position d'extrémité et les polymères organiques contenant des groupes silane.

11. Composition durcissable contenant au moins une amidine de formule (I) selon l'une quelconque des revendications 1 à 5.

12. Composition selon la revendication 11, **caractérisée en ce qu'**elle contient en outre au moins un polymère contenant des groupes silane.

13. Composition selon l'une quelconque des revendications 11 à 12, **caractérisée en ce qu'**elle représente un adhésif ou un matériau d'étanchéité ou un revêtement.
